# EUROPEAN PATENT SPECIFICATION

(11) **EP 0 251 449 B1**
(45) Date of publication and mention of the grant of the patent: **01.02.1995**
(21) Application number: 87303919.2
(22) Date of filing: 30.04.1987
(51) Int. Cl.: C12N 15/12, C12P 21/02, A61K 38/00, C07K 14/00

(54) **Recombinant alveolar surfactant protein**
Rekombinantes Alveolar-oberflächenaktives Protein
Protéine recombinante surfactante des alveoli

(30) Priority: 30.04.1986 US 857715
(43) Date of publication of application: 07.01.1988
(73) Proprietor: SCIOS NOVA INC., Mountain View, CA 94043 (US)
(72) Inventor: Schilling, James W., Palo Alto California (US); White, Robert T., Fremont California 94538 (US); Cordell, Barbara, San Francisco California 94109 (US); Benson, Bradley J., San Francisco California 94127 (US)
(74) Representative: Goldin, Douglas Michael

(56) References cited:
- WO-A-86/03408
- Proc. Natl. Acad. Sci. USA, vol. 84, Jan. 1987, pp. 66-70, Biochemistry, S. Hawgood et al.
- Biological Abstracts/RRM, abstract 33040847, Philadelphia, US, R. Warr et al.

## Description

The invention relates to the field of recombinant protein production. More specifically it relates to the production of various forms of alveolar surfactant protein (ASP) which are useful in the management of certain respiratory diseases.

The human lung is composed of a large number of small sacs or alveoli in which gases are exchanged between the blood and the air spaces of the lung. In healthy individuals, this exchange is mediated by the presence of a protein containing surfactant complex which is synthesized in the microsomal membranes of type II alveolar cells. In the absence of adequate levels of this complex, a lung cannot properly function--i.e., the alveoli collapse during exhalation, and cannot be subsequently re-inflated by inhaling. Thus, the untreated inability to synthesize this complex may result in death or in severe physical damage.

The best documented instance of inadequate surfactant complex levels occurs in premature infants and infants born after complicated pregnancies, and is widely known as respiratory distress syndrome (RDS) . A widely publicized form of this syndrome has been designated hyaline membrane disease, or idiopathic RDS. RDS is currently the leading cause of infant mortality and morbidity in the United States and in other developed countries, and substantial efforts have been directed to diagnosis and treatment. Current treatment has focused on mechanical (pressure) ventilation which, at best, is an invasive stop-gap measure that often results in damage to the lung and other deleterious side effects, including complications such as bronchopulmonary dysplasia, interstitial emphysema and pneumothorax. Mental retardation has also resulted on occasion when this treatment was used (Hallman, M., et al, Pediatric Clinics of North America (1982) 29: 1057-1075).

Limited attempts have been made to treat the syndrome by surfactant substitution. This would be a method of choice, as, in general, only one administration is required, and the potential for damage is reduced. For example, Fujiwara, et al, Lancet (1980) 1:55-used a protein-depleted surfactant preparation derived from bovine lungs; the preparation is effective but immunogenic. Hallman, M., et al, Pediatrics (1983) 71:473-482 used a surfactant isolate from human amniotic fluid to treat a limited number of infants with some success. U.S. Patent 4,312,860 to Clements discloses an artificial surfactant which contains no protein and is said to be useful in this approach although no data are shown. In short, surfactant substitution has not been widely used clinically.

The preferred surfactant substitute would be the lung surfactant complex itself. This complex is composed of apoprotein, two phospholipids (dipalmitoyl phosphocholine (DPPC) and phosphatidyl-glycerol (PG)) which are present in major amount, several lipid components present in only very minor amount, and calcium ions. The apoprotein contains proteins having molecular weights of the order of 32,000 daltons and very hydrophobic proteins of the order of about 10,000 daltons (King, R.J. et al, Am J Physiol (1973) 224:788-795). The 32,000 dalton protein is glycosylated and contains hydroxyproline.

A major reason for the limited progress in surfactant replacement therapy has been the lack of availability of the protein portion of the complex. Replacement therapies have focused on attempts to use the lipid components alone, and it appears that the performance of such treatment can be markedly improved by addition of the apoprotein (Hallman, M., et al, Pediatric Clinics of North America (1982) (supra)). At present, however, several of these proteins are available only from normal adult human lung, and from amniotic fluid. Even efficient isolation procedures would not provide an adequate supply. Thus, it would be desirable to have available a method for producing practical quantities of apoprotein for use alone or in conjunction with the saturated phospholipid portion of the complex.

PCT patent application WO86/03408, describes the recombinant production of the human ASP protein of about 32 kd, the retrieval of DNA sequences encoding various canine ASP proteins and the retrieval of a single representative of the human ASP protein group of about 10 kd molecular weight. It is now clear that efficient production of the "10K" group is required for use in adequate therapy.

The invention provides a means for obtaining additional members of the apoprotein portion of the lung surfactant complex in quantity and under conditions which permit optimization of its features. The remaining components of the complex, dipalmitoyl phosphatidylcholine and phosphatidylglycerol, along with calcium ions are already readily available, as are certain members of the ASP group as described in WO86/03408 (supra). The availability of required quantities of the additional forms of apoprotein makes possible research efforts to optimize the form of complex useable in therapy, and opens the possibility for routine replacement therapy of respiratory distress syndrome.

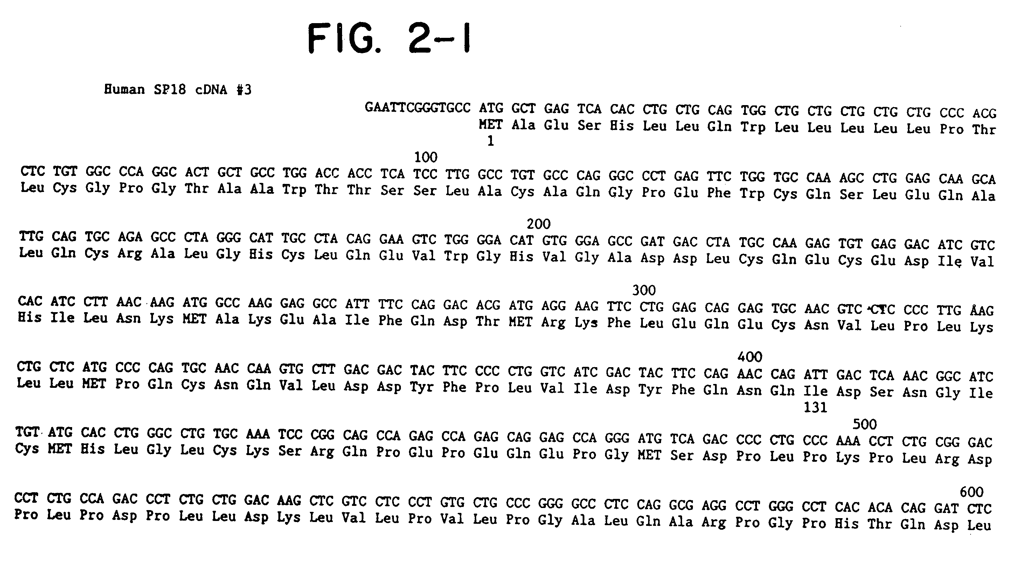

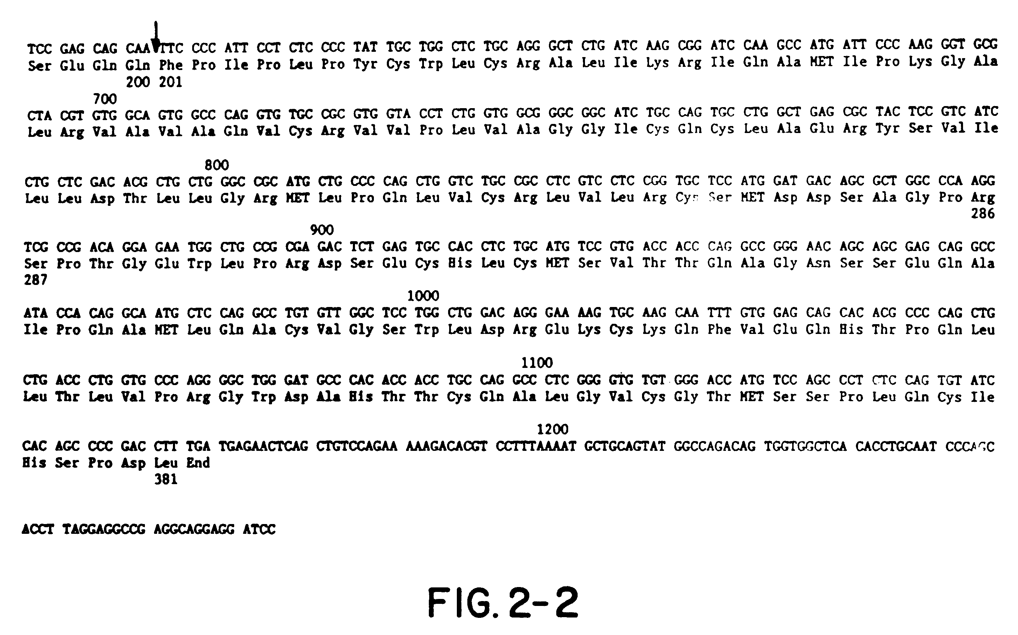

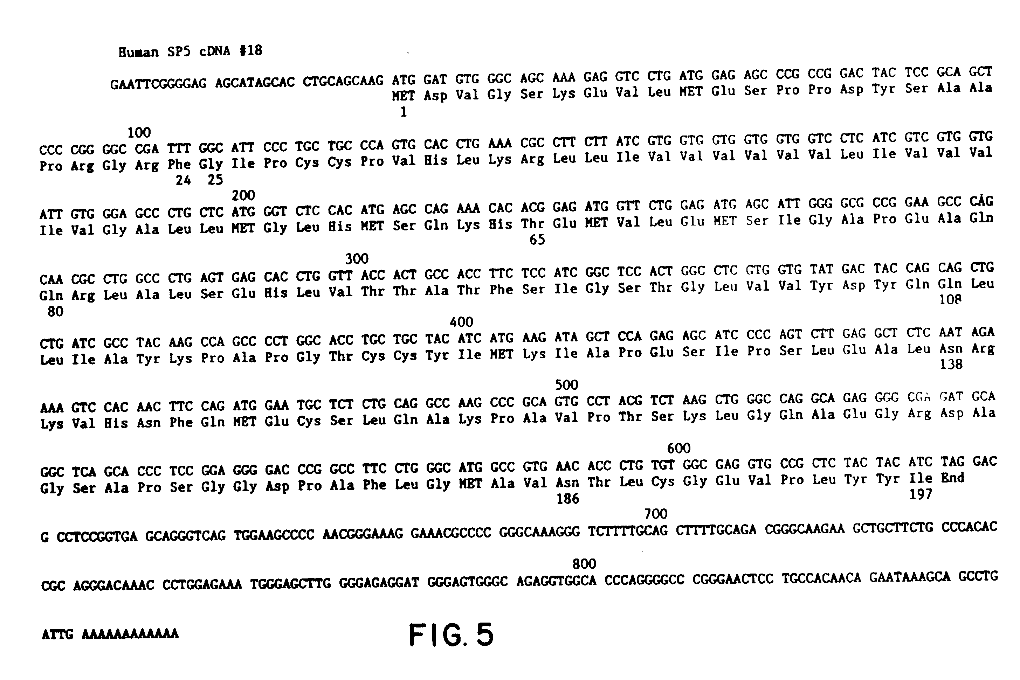

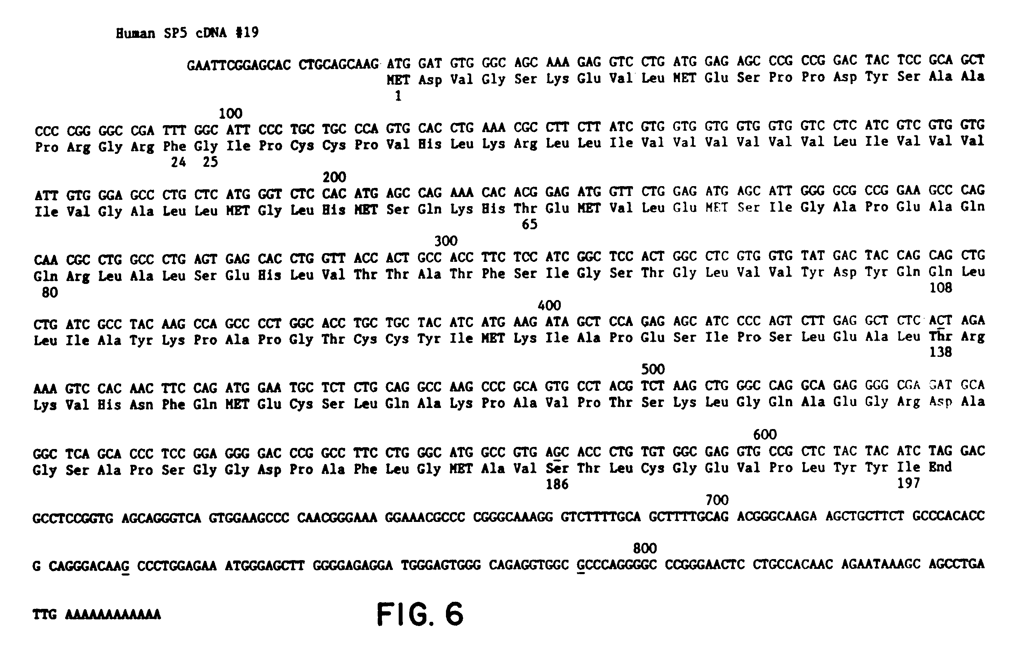

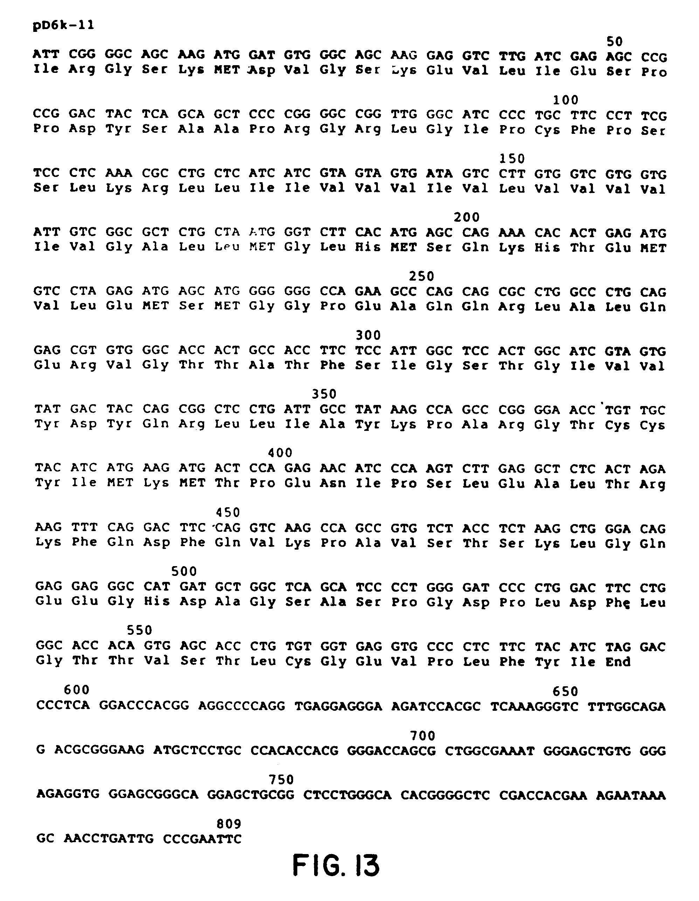

The present invention relates to an alveolar surfactant protein (ASP) free of proteins normally accompanying it in situ, which is
(a) isolated and purified human ASP having the amino acid sequence of the mature protein encoded by the DNA shown in Figure 2, having an amino terminus at position 201 of said figure or encoded by a naturally occurring allelic variant thereof;
(b) isolated and purified human ASP having the amino acid sequence of the mature protein encoded by the DNA shown in Figure 5 or Figure 6, and having a molecular weight of approximately 5 kd, or encoded by a naturally occurring allelic variant thereof; or
(c) isolated and purified canine ASP having the amino acid sequence of the mature protein encoded by the DNA shown in Figure 13, having a molecular weight of approximately 5 kd, or encoded by a naturally occurring allelic variant thereof.

Relatively high molecular weight, relatively water soluble proteins of about 32kd (32K ASP) have been disclosed as described above. Lower molecular weight, hydrophobic proteins of about 5-20kd (10K ASP) are described herein. Both groups of proteins encourage formation of surface tension lowering films when complexed with phospholipid in the presence of calcium ion. However, in vivo studies show that the 10K group and individual members thereof are dramatically more effective than 32K ASP in obtaining and maintaining inflation of the lungs and that the combination of 10K and 32K proteins is synergistic.

The invention further relates to expression vectors containing DNA encoding the proteins of the invention, which are suitable for production of the proteins, to recombinant host cells transformed with these vectors, and to methods of producing the recombinant ASPs by culturing such cells. In another aspect the invention relates to pharmaceutical compositions effective in treating RDS in mammals comprising a protein of the invention in admixture with a phospholipid preparation and optionally with a pharmaceutically acceptable excipient.

Figure 1 shows the DNA sequence (along with the deduced amino acid sequence) determined for cDNA encoding a canine 18 kd ASP protein from overlapping cDNA clones, showing the overlapping pD10k-1 and pD10k-4 clones identified.

Figure 2 shows cDNA sequence and deduced amino acid sequence for "cDNA No. 3" encoding human 18 kd ASP protein.

Figure 3 shows the DNA sequence and deduced amino acid sequence of the exon portions of the genomic DNA encoding human 18 kd protein.

Figure 4 shows the sequence of oligonucleotide probes used to isolate the cDNA encoding human 5 kd/8 kd protein.

Figure 5 shows the DNA and deduced amino acid sequence of cDNA No. 18 encoding human 5 kd protein.

Figure 6 shows an analogous cDNA No. 19 encoding human 5 kd protein.

Figures 7a and 7b are results of SDS PAGE without and with endo F enzyme treatment of ³⁵S labeled proteins produced in CHO cells transfected with vectors encoding human 18 kd protein.

Figure 8 shows an SDS gel obtained from bacteria transfected with expression vectors for human 18 kd protein (and controls) labeled with ³⁵S methionine.

Figure 9 shows a Western blot of bacterial extracts corresponding to those of Figure 8.

Figure 10 shows the results of an in vitro determination of the ability of various ASP proteins to enhance surface tension-lowering by phospholipids.

Figure 11 shows the results of an additional in vitro determination of the ability of human 18 kd and 5 kd proteins to enhance surface tension lowering by phospholipids.

Figure 12 shows the results corresponding to those of Figure 11 for the canine proteins, with and without the addition of 32 kd protein.

Figure 13 shows the nucleotide sequence of a canine SP-5 cDNA clone.

Figure 14 shows a comparison of the amino acid sequences encoded by two cDNA clones obtained from a human lung library in λgt10, as well as that encoded by the genomic clone described as gHS-15 in WO86/03408. Also shown are the sequences encoded by two cDNAs recovered by others.

Figure 15 shows the nucleotide sequence of a synthetic trp promoter used for bacterial expression of the surfactant proteins.

### A. Definitions

As used herein, "alveolar surfactant protein (ASP)" refers to apoprotein associated with the lung surfactant complex and having ASP activity as defined hereinbelow. The ASP of all species examined appears to comprise one or more components of relatively high molecular weight (of the order of 32 kd) designated herein "32K ASP" and one or more quite hydrophobic components of relatively low molecular weight (of the order of 5-20 kd) designated herein "10K ASP'. (King, R.J., et al, J Appl Physiol (1977) 42:483-491; Phizackerley, P.J.R., Biochem J (1979) 183:731-736.)

The 32K proteins for all species appear to be derived from a single primary amino acid sequence, in each case (although there is evidence that the protein is encoded by multiple genes encoding proteins with minor variations in sequence). The multiple components, found under some conditions, however, of differing molecular weights, are due to variations in glycosylation patterns. WO86/03408, discloses the complete amino acid sequence for the human and canine 32K ASP proteins which show a high degree of homology. This set of high molecular weight, relatively hydrophilic proteins and the 32K ASP derived from alternate mammalian species is expected to exhibit a high degree of homology with the canine and human sequences presented.

The low molecular weight "10K" proteins are relatively hydrophobic and also appear to be mixtures of several proteins of varying molecular weight. Both the human and canine proteins exhibit unreduced molecular weights of 18 kd, 8 kd, and 5 kd. The 8 kd and 5 kd proteins appear to be identical in N-terminal sequence and are presumably derived from the same message but contain variations in C-terminal processing. The 18 kd protein, which shows a molecular weight of 10 kd under reducing conditions, on the other hand, has a clearly different amino acid sequence. However, the 18 kd, 8 kd and 5 kd proteins of the mammalian species concerned herein, all appear to function equivalently in vivo. The invention herein concerns this 10K group. WO86/03408, disclosed the complete cDNA and deduced amino acid sequence for the 18 kd canine protein, but only a partial DNA sequence for the human counterpart. Only a short N-terminal amino acid sequence for the 8 kd/5 kd canine protein was disclosed; the appropriate cDNA has now been recovered for the human protein and the complete sequence of both representative 10K proteins made part of the art. Because the 10K mixture seems to show products of only two DNA sequences, although variations in posttranslational processing can result in multiple molecular weights, the designations SP-18 and SP-5 have been adopted for these two types of proteins and genes.

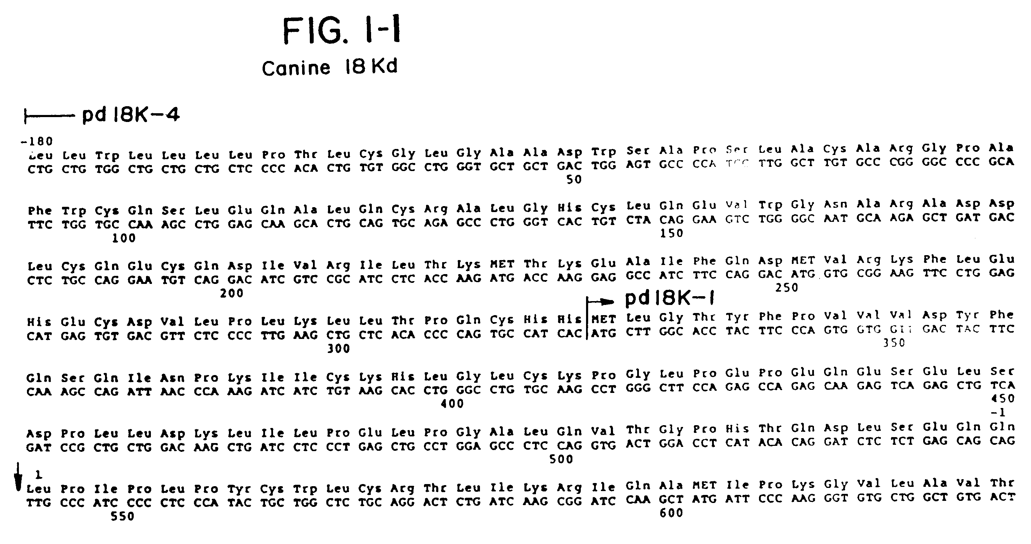

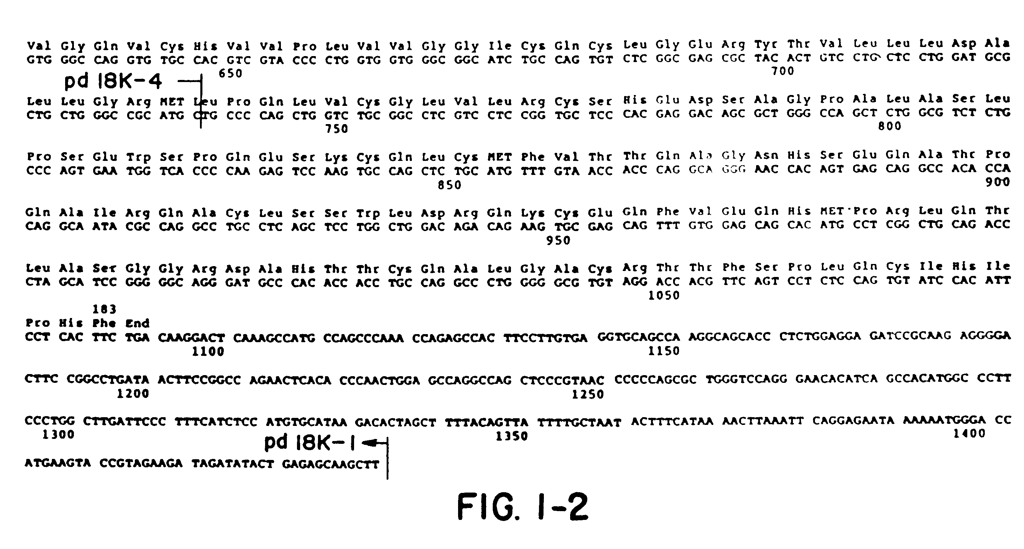

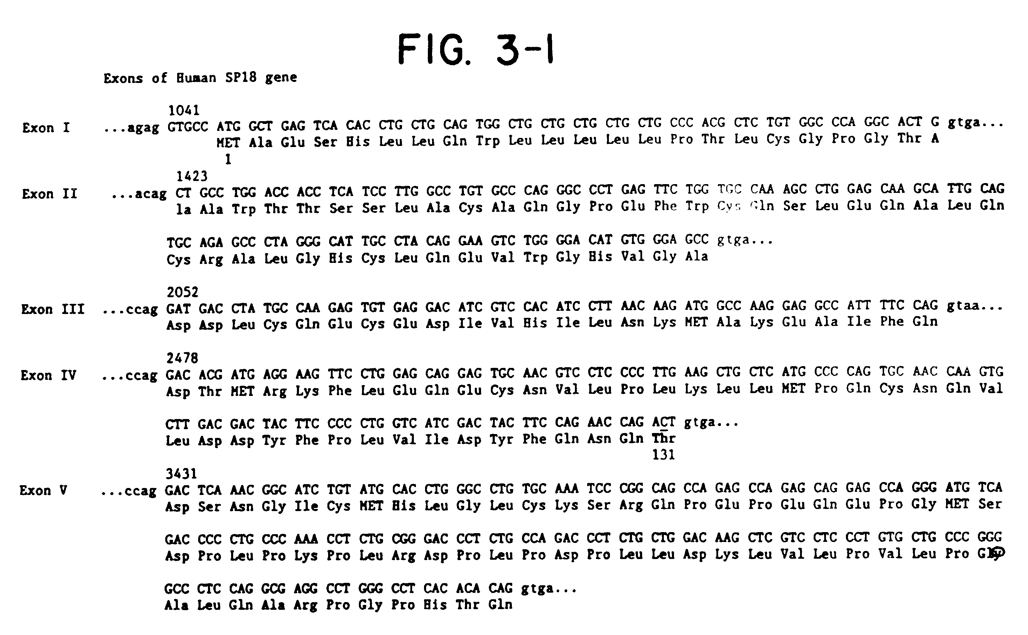

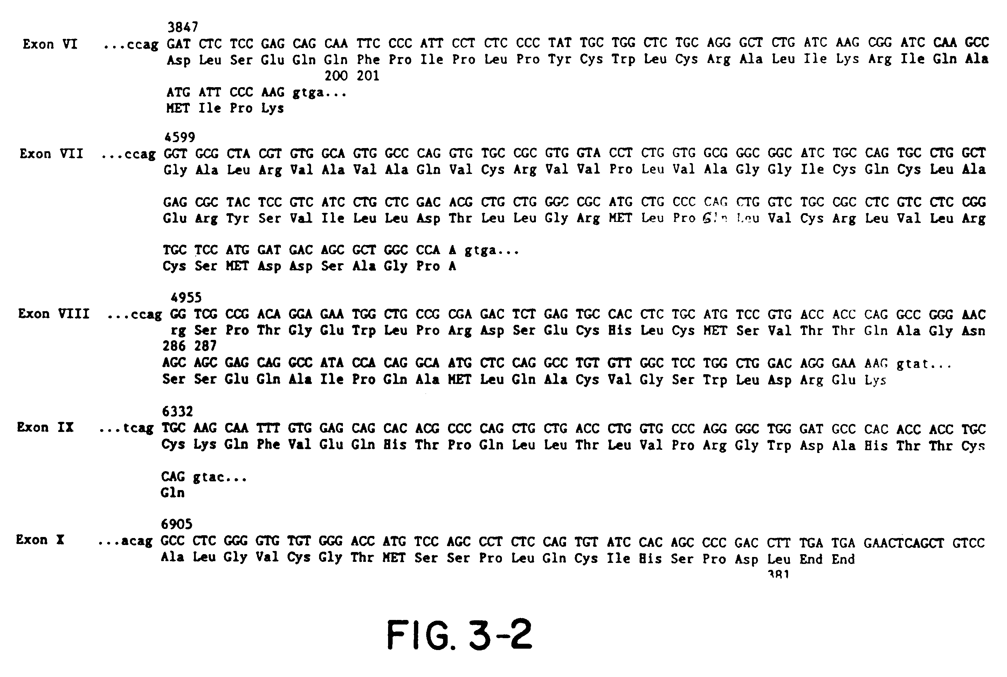

Figure 1 herein corresponds to Figure 2 of WO86/03408 and shows the complete cDNA sequence for the mature canine SP-18 protein beginning at leucine shown at position 1 and ending at phenylalanine at position 183. The corresponding sequence for the human SP-18 protein is shown in Figures 2 and 3, sequences which differ only slightly in amino acid sequence as described hereinbelow. The start of the mature protein is the phenylalanine residue at position 201 of Figure 2 ending with the leucine at position 381. The cDNA thus putatively encodes a 181 amino acid protein for the human. Both the human and dog proteins are, however, thought to be processed to shorter sequences by deletion of a portion of the carboxy-terminal sequence. For the human protein, this is thought to occur so that the secreted protein terminates with the arginine shown at position 286 in Figure 2. Such processing would result in a protein of molecular weight about 10K seen in reduced electrophoresis gels of isolated mature protein.

The cDNA and deduced amino acid sequences for two analogous forms of human SP-5 protein are shown in Figures 5 and 6. Again, although the cDNA, starting at the putative N-terminus of the mature protein encodes 173 or 174 amino acids, variations in C-terminal processing results in isolated proteins of 5 kd or 8 kd.

In summary, the 10K group of lower molecular weight proteins appears to derive from DNAs encoding two different species designated herein SP18 and SP5. The SP18 encoded species are so named because they encode a putative mature protein of approximately 18 kd; however, post-translational processing appears to result in proteins of lower molecular weight. (Coincidentally, the approximately 20 kd protein dimer resulting from the processed protein under reducing conditions runs in gels at the position expected for an 18 kd protein.) Similarly, SP5 encodes a protein of putative molecular weight of approximately 19 kd. However, again, this molecular weight protein is not found in extracts, and the encoded amino acid sequence is evidently processed to the 5 kd and 8 kd proteins obtained.

The recombinant ASP proteins of the invention have amino acid sequences corresponding to those illustrated herein. It is understood that limited modifications may, however, occur to provide naturally occurring allelic variant without destroying activity. For example, the human ASP SP18 recombinant protein of the invention has an amino acid sequence substantially similar to that shown in Figure 2, but naturally occurring minor modifications of this sequence which do not destroy activity also fall within the definition of SP18 human ASP and within definition of the protein claimed as such, as further set forth below.

As is the case for all proteins, the ASP proteins can occur in neutral form or in the form of basic or acid addition salts depending on its mode of preparation, or, if in solution, upon its environment. It is well understood that proteins in general, and, therefore, any ASP, in particular, may be found in the form of its acid addition salts involving the free amino groups, or basic salts formed with free carboxyls. Pharmaceutically acceptable salts may, indeed, enhance the functionality of the protein. Suitable pharmaceutically acceptable acid addition salts include those formed from inorganic acids such as, for example, hydrochloric or sulfuric acids, or from organic acids such as acetic or glycolic acid. Pharmaceutically acceptable bases include the alkali hyroxides such as potassium or sodium hydroxides, or such organic bases as piperidine, glucosamine, trimethylamine, choline, or caffeine. In addition, the protein may be modified by combination with other biological materials such as lipids and saccharides, or by side chain modification, such as acetylation of amino groups, phosphorylation of hydroxyl side chains, or oxidation of sulfhydryl groups or other modification of the encoded primary sequence. Indeed, in its native form, ASP proteins are glycosylated, and certain of the encoded proline residues have been converted to hydroxyproline. The proteins are also found in association with the phospholipids in particular DPPC and PG. Included within the definition of any ASP protein form herein are glycosylated and unglycosylated forms, hydroxylated and non-hydroxylated forms, the apoprotein alone, or in association with lipids. Such modified forms retain their ability to facilitate the exchange of gases between the blood and lung air spaces and to permit re-inflation of the alveoli.

It is further understood that minor naturally occurring modifications of primary amino acid sequence may result in proteins which have substantially equivalent or enhanced activity as compared to any particular illustrated sequence.

"ASP activity" for a protein is defined as the ability, when combined with lipids either alone or in combination with other proteins, to exhibit activity in the in vivo assay of Robertson, B. Lung (1980) 158: 57-68, and described hereinbelow. In this assay, the sample to be assessed is administered through an endotracheal tube to fetal rabbits or lambs delivered prematurely by Caesarian section. (These "preemies" lack their own ASP, and are supported on a ventilator.) Measurements of lung compliance, blood gases and ventilator pressure provide indices of activity. Preliminary assessment of activity may also be made by an in vitro assay, for example that of King, R. J., et al, Am J Physiol (1972) 223:715-726, or that illustrated below of Hawgood, et al, which utilizes a straightforward measurement of surface tension at a air-water interface when the protein is mixed with a phospholipid vesicle preparation. The 10K and 32K ASP proteins described herein show ASP activity in combination as well as independently. Although it had previously been believed that the 10K protein displayed ASP activity only when acting in concert with the 32K family, the inventors herein have now demonstrated that the 10K protein alone displays significant ASP activity and that supplementation with the 32K protein acts synergistically to enhance activity of the 10K protein(s).

"Operably linked" refers to a juxtaposition wherein the components are configured so as to perform their usual function. Thus, control sequences operably linked to coding sequences are capable of effecting the expression of the coding sequence.

"Control sequence" refers to a DNA sequence or sequences which are capable, when properly ligated to a desired coding sequence, of effecting its expression in hosts compatible with such sequences. Such control sequences include promoters in both procaryotic and eucaryotic hosts , and in procaryotic organisms also include ribosome binding site sequences, and, in eucaryotes, termination signals. Additional factors necessary or helpful in effecting expression may subsequently be identified. As used herein, "control sequences" simply refers to whatever DNA sequence may be required to effect expression in the particular host used.

"Cells" or "recombinant host cells" or "host cells" are often used interchangeably as will be clear from the context. These terms include the immediate subject cell, and, of course, the progeny thereof. It is understood that not all progeny are exactly identical to the parental cell, due to chance mutations or differences in environment. However, such altered progeny are included when the above terms are used.

### B. General Description

The methods illustrated below to obtain DNA sequences encoding ASP are merely for purposes of illustration and are typical of those that might be used. However, other procedures may also be employed, as is understood in the art.

### B.1. The Nature of the Surfactant Complex

The alveolar surface of lung has been studied extensively by a number of techniques, and by a number of groups. It appears that the membrane of the alveolus is composed of type I and type II alveolar cells, of which the type II cells comprise approximately 3% of the surface. The type II cells are responsible for the exocrine secretion of materials into a lining fluid layer covering the basement membrane, which materials decrease the surface tension between the liquid of the lining and the gas phase of the contained volume. The fluid layer, then, is comprised of water derived from the blood plasma of the alveolar capillaries, and the surfactant secretions of the type II cells.

The type II cells, themselves, contain 60-100 pg of protein and about 1 pg of lipid phosphorus per cell where the ratio between type II cell DPPC and PG phosphorus is about 8 to 1. Studies of the apoprotein components have been based on pulmonary lavage from various species, and have been shown to comprise two major protein types, as discussed above, of approximate molecular weights 10-20 kd and of 32 kd (Kikkawa, Y., et al, Laboratory Investigation (1983) 49:122-139.) It is not clear whether the apoproteins are bound to the phospholipid component (King, R. J., et al, Am Rev Respir Dis (1974) 110:273) or are not (Shelly, S. A., et al, J Lipid Res (1975) 16:224).

It has been shown that the higher molecular weight protein obtained by pulmonary lavage of dogs, and separated by gel electrophoresis is composed of 3 major components of molecular weight 29,000, 32,000, and 36,000 daltons. The 32,000 dalton protein was used to obtain sequence data, as set forth below; however, all 3 of these proteins have identical N-terminal sequences. and there is evidence that they differ only in degree of glycosylation. Digestion of the 36 kd and 32 kd bands with endoglycosidase F, which removes carbohydrate side chains, results in products which co-migrate with the 29 kd component. The mobility of the 29 kd component is unaffected by this treatment. It has also been shown that the 32 kd fraction aggregates into dimers and trimers.

The smaller molecular weight proteins are extracted with more difficulty, but these, too, appear to be mixtures (Phizackerley et al., supra; description below). For both the dog and human proteins, which have been studied with respect to their encoding DNA, and with respect to bovine lavage, studied at the protein level, the lower molecular weight protein mixtures appear to contain two types of amino acid sequence, designated herein SP-18 and SP-5. The SP-18 sequences are encoded by cDNA corresponding to a molecular weight primary sequence of approximately 18 kd; approximately 180 amino acids. However, the products appears to be processed in vivo to shorter proteins. The SP-5 DNA encodes a mature protein of approximately 173 amino acids, but this protein, too, is processed to substantially smaller proteins apparently of approximately 5 kd and 8 kd. The processing referred to above seems to comprise deletion of sequences from the C-terminus of the proteins produced.

### B.2. Cloning of Coding Sequences for Canine and Human ASP Proteins

The entire canine and human ASP 32K protein encoding sequences have been cloned and expressed as set forth in WO86/03408. Herein, DNA sequences encoding several of the lower molecular weight proteins from both human and canine sources have also been obtained and expressed.

The canine lung cDNA library was probed with two synthetic oligomer mixtures designed to correspond to the N-terminal amino acid sequence of an 18 kd (on unreduced gels) canine protein, and clones hybridizing to both probes were recovered and sequenced; this provided the information set forth in Figure 1 herein. One of these clones, which contained canine ASP encoding sequence, was used to probe a cDNA library prepared in bacteriophage λgt10 from mRNA isolated from adult human lung to obtain a human SP-18; which was, in turn, used to probe a human genomic library. The complete sequence(s) for human SP-18 encoded by the cDNA and by the genomic clone are disclosed. Probes designed corresponding to the N-terminal amino acid sequence of a 5 kd canine protein were then used to obtain SP-5 cDNA from the λgt10 lung library. Variants of this sequence are also disclosed.

### B.3. Expression of ASP

As the nucleotide sequences encoding the additional human and canine ASP proteins are now available, these may be expressed in a variety of systems. If procaryotic systems are used, an intronless coding sequence should be used, along with suitable control sequences. The cDNA clones for any of the above ASP proteins may be excised with suitable restriction enzymes and ligated into procaryotic vectors for such expression. For procaryotic expression of ASP genomic DNA, the DNA should be modified to remove the introns, either by site-directed mutagenesis, or by retrieving corresponding portions of cDNA and substituting them for the intron-containing genomic sequences The intronless coding DNA is then ligated into expression vectors for procaryotic expression. Several illustrative expression systems are set forth below.

As exemplified below. ASP encoding sequences may also be used directly in an expression system capable of processing the introns, usually a mammalian host cell culture. To effect such expression, the genomic sequences can be ligated downstream from a controllable mammalian promoter which regulates the expression of these sequences in suitable mammalian cells.

In addition to recombinant production, proteins of the invention of sufficiently short length, such as the 5 kd protein, may be prepared by protein synthesis methods.

### B.4. Protein Recovery

The ASP protein may be produced either as a mature protein or a fusion protein, or may be produced along with a signal sequence in cells capable of processing this sequence for secretion. It is advantageous to obtain secretion of the protein, as this minimizes the difficulties in purification; thus it is preferred to express the human ASP gene which includes the codons for native signal sequence in cells capable of appropriate processing. It has been shown that cultured mammalian cells ore able to cleave and process heterologous mammalian proteins containing signal sequences, and to secrete them into the medium (McCormick, F., et al, Mol Cell Biol (1984) 4:166).

When secreted into the medium, the ASP protein is recovered using standard protein purification techniques. The purification process is simplified, because relatively few proteins are secreted into the medium, and the majority of the secreted protein will, therefore, already be ASP. However, while the procedures are more laborious, it is within the means known in the art to purify this protein from sonicates or lysates of cells in which it is produced intracellularly in fused or mature form.

### B.5. Assay for ASP Activity

In vitro methods have been devised to assess the ability of ASP proteins to function by reducing surface tension (synonymous with increasing surface pressure) to generate a film on an aqueous/air interface. Studies using these methods have been performed on the isolated native 32K canine ASP. (Benson, B.J., et al Prog Resp Res (1984) 18:83-92; Hagwood, S., et al, Biochemistry (1985) 24:184-190.)

Tanaka, Y, et al, Chem Pharm Bull (1983) 31:4100-4109 disclose that a 35 kd protein obtained from bovine lung enhanced the surface spreading of DPPC; Suzuki, Y., J Lipid Res (1982) 23:62-69; Suzuki, Y., et al, Prog Resp Res (1984) 18:93-100 showed that a 15 kd protein from pig lung enhanced the surface spreading of the lipid-protein complex from the same source.

Since the function of the surfactant complex in vivo is to create a film at the air/aqueous interface in order to reduce surface tension, the ability of ASP proteins to enhance the formation of the film created by the spread of lipid or lipoprotein at such a surface in an in vitro model is clearly relevant to its utility.

An in vivo model, described in the examples, may also be employed.

### B.6. Administration and Use

The purified proteins can be used alone and in combination in pharmaceutical compositions appropriate for administration for the treatment of respiratory distress syndrome in infants or adults. The compositions and protein products of the invention are also useful in treating related respiratory diseases such as pneumonia and bronchitis. The complex contains about 50% to almost 100% (wt/wt) lipid and 50% to less than 1% ASP; preferably ASP is 5%-20% of the complex. The lipid portion is preferably 80%-90% (wt/wt) DPPC with the remainder unsaturated phosphatidyl choline, phosphatidyl glycerol, triacylglycerols, palmitic acid or mixtures thereof. The complex is reassembled by mixing a solution of ASP with a suspension of lipid liposomes, or by mixing the lipid protein solutions directly in the presence of detergent or an organic solvent. The detergent or solvent may then be removed by dialysis.

While it is possible to utilize the natural lipid component from lung lavage in reconstructing the complex, arid to supplement it with appropriate amounts of ASP proteins, the use of synthetic lipids is clearly preferred. First, there is the matter of adequate supply, which is self-evident. Second, purity of preparation and freedom from contamination by foreign proteins, including infectious proteins, which may reside in the lungs from which the natural lipids are isolated, are assured only in the synthetic preparations. Of course, reconstitution of an effective complex is more difficult when synthetic components are used.

As noted above, it had been previously been believed that the 10K ASP mixture served primarily to enhance the activity of the 32K mixture; however, it has now been established by the inventors herein that a preferred composition comprises either a complex with the 10K protein alone, the SP-5 or SP-18 protein alone, a complex of the 10K arid 32K mixtures, or a complex of an SP-18 or SP-5 protein and the 32K mixture. In the latter two cases, a preferred protein ratio -- i.e., 32K:10K or 32K:SP-18 or 32K:SP-5 -- is typically in the range of 3:1 to 200:1, preferably about 10:1 to 5:1. The 32K protein may be added directly to an aqueous suspension of phospholipid vesicles in an aqueous solution. Because it is so hydrophobic, the 10K mixture (or the SP-5 or the SP-18 proteins) is added to the lipids in an organic solvent, such as chloroform, the solvents evaporated, and the vesicles re-formed by hydration.

The addition of the 32K protein to the 10K type for the administration of the surfactant complex appears to have a synergistic effect--i.e., the combination of 32K and 10K type proteins exerts the desired activity at protein concentrations lower than those required for the 10K protein alone. Accordingly, in a preferred method of the invention, the surfactant complex administered will contain an effective amount of the 10K mixture, or of the individual SP-5 or SP-18 proteins in admixture with the 32K ASP. Particularly preferred compositions contain the ratios of 32K:10K type protein as set forth above, along with a suitable amount of lipid component, typically in the range of 50 - almost 100% of the composition.

The compositions containing the complex are preferably those suitable for endotracheal administration, i.e., generally as a liquid suspension, as a dry powder "dust" or as an aerosol. For direct endotracheal administration, the complex is suspended in a liquid with suitable excipients such as, for example, water, saline, dextrose, or glycerol and the like. The compositions may also contain small amounts of nontoxic auxiliary substances such as pH buffering agents, for example, sodium acetate or phosphate. To prepare the "dust", the complex, optionally admixed as above, is lyophilized, and recovered as a dry powder.

If to be used in aerosol administration, the complex is supplied in finely divided form along with an additional surfactant and propellent. Typical surfactants which may be administered are fatty acids and esters, however, it is preferred, in the present case, to utilize the other components of the surfactant complex, DPPC and PG. Useful propellents are typically gases at ambient conditions, and are condensed under pressure. Lower alkanes and fluorinated alkanes, such as Freon, may be used. The aerosol is packaged in a container equipped with a suitable valve so that the ingredients may be maintained under pressure until released.

The surfactant complex is administered, as appropriate to the dosage form, by endotracheal tube, by aerosol administration, or by nebulization of the suspension or dust into the inspired gas. Amounts of complex between about 0.1 mg and 200 mg, preferably 50-60 mg/kg body weight, are administered in one dose. For use in newly born infants, one administration is generally sufficient. For adults, sufficient reconstituted complex is administered to replace demonstrated levels of deficiency (Hallman. M., et al, J Clinical Investigation (1982) 70:673-682).

### C. Standard Methods

Most of the techniques which are used to transform cells, construct vectors, extract messenger RNA, prepare cDNA libraries, and the like are widely practiced in the art, and most practitioners are familiar with the standard resource materials which describe specific conditions and procedures. These methods are set forth with particularity in WO86/03408.

As set forth in this predecessor application, expression may be achieved in a variety of host systems including, in particular, mammalian and bacterial systems, as well as yeast based systems. In addition, other cell systems have become available in the art, such as the baculovirus vectors used to express protein encoding genes in insect cells. The expression systems set forth below are illustrative, and it is understood by those in the art that a variety of expression system can be used.

### D. Examples

### D.1. Isolation of Mammalian ASP Proteins

Canine, human and bovine ASP proteins were obtained in purified form.

### D.1.a. Isolation of the Canine Surfactant Complex

Lung surfactant complex was prepared from canine lungs obtained from exsanguinated canines. All procedures, including the lavage, were performed at 4°C and the isolated material was stored at -15°C.

The lungs were degassed and lavaged 3 times with one liter per lavage of 5 mM Tris-HCl, 100 mM NaCl, pH 7.4 buffer. The Ca⁺² concentration of this buffer was less than 5 x 10⁻⁶ M (Radiometer F2112 Ca; Radiometer A/S, Copenhagen, Denmark). The pooled lung washings were spun at 150 x gₐᵥ for 15 min (Sorval RC2-B) to remove cellular material. The supernatant was then spun at 20,000 x gₐᵥ for 15 hr (Beckman L3-40) using a type 15 rotor (Beckman Instruments), and the resulting pellet was dispersed in buffer containing 1.64 M sodium bromide. After equilibration for 1 hr, the suspension was spun at 100,000 x gₐᵥ for 4 hr (Beckman L5-50B) in a SW28 rotor (Beckman Instruments) . The pellicle was resuspended in buffer and spun at 100,000 x gₐᵥ for 1 hr (Beckman L5-50B). This pellet containing the complex was resuspended in double distilled water.

Pellet resuspended in water at a concentration of 10-15 mg phospholipid/ml was injected into a 50-fold volume excess of n-butanol (Sigrist, H., et al, Biochem Biophys Res Commun (1977) 74:178-184) and was stirred at room temperature for 1 hr. After centrifugation at 10.000 x gₐᵥ for 20 min (Sorval RC2-B), the pellet, which contains the 32K ASP is recovered for further purification as described below. The supernatant, which is a single phase, contains the lipids and the lower molecular weight proteins. To obtain the lipids, the supernatant was dried under vacuum at 40°C and the lipids were extracted (Folch, J., et al, J Biol Chem (1957) 226:497-509).

To obtain the hydrophobic protein, the supernatant was subjected to Rotovap to remove the butanol, and further dried by addition of ethanol followed by Rotovap. The dried residue was suspended in redistilled chloroform containing 0.1 N HCl, and insoluble material removed by centrifugation.

The resulting solution was chromatographed over an LH-20 column (Pharmacia) and developed in chloroform. (LH-20 is the hydroxypropyl derivative of Sephadex G-50; it is a hydrophobic gel which is inert to organic solvents.) The proteins are excluded; lipids/phospholipids elute from the included volume.

Protein was recovered from the void volume fractions by evaporation of the chloroform under nitrogen, and then subjected to sizing on polyacrylamide gels. When run under non-reducing conditions, bands of approximately 18 kd (identified in WO86/03408 as 16.5 kd), 8 kd (identified in WO86/03408 as 12 kd), and 5 kd (identified in WO86/03408 as 6 kd) were obtained; under reducing conditions, a single broad band of 5-12 kd was found.

The 18 kd, 8 kd, and 5 kd bands from the non-reduced gels were subjected to N-terminal analysis by Edman degradation, to give the following sequences: 
For 18 kd:   ?-Pro-Ile-Pro-Leu-Pro-Tyr-Cys-Trp-Leu-Cys-
Arg-Thr-Leu-Ile-Lys-Arg-Ile-Gln-Ala-Met-Ile-
Pro-Lys-Gly-Val-Leu-Ala-Val-Thr- ? -Gly-Gln-

For 8 kd:   Ile-Pro-Cys-Phe-Pro-Ser-Ser-Leu-Lys-Arg-Leu-
Leu-Ile-Ile-Val-Trp-

For 5 kd:   Ile-Pro-Cys-Phe-Pro-Ser-Ser-Leu-Lys-Arg-Leu-
Leu-Ile-Ile-Val-Trp-
The 5-12 kd band also represents a mixture of the 18 kd, 8 kd and 5 kd proteins, designated herein as the "10K" mixture of proteins.

The precipitate from the n-butanol extraction above was used to obtain the purified 32K apoprotein as described in WO86/03408 (supra).

### D.1.b. Isolation of Human ASP

Human 32K and lower molecular weight ASP was prepared following the procedure described in the published WO86/03408.

The isolated low molecular weight hydrophobic proteins show bands corresponding to 18 kd, 8 kd and 5 kd when subjected to polyacrylamide gel electrophoresis under non-reducing conditions. Under reducing conditions, a single broad band corresponding to 5-12 kd is obtained. The molecular weights of these bands are slightly different from those reported in the published application.

### D.1.c. Isolation of Bovine ASP

The 10K bovine ASP containing 5 kd and 18 kd proteins was isolated from the lavage fluid of bovine lungs, in a method similar to that used for canine ASP.

Excised bovine lungs were filled with Tris-buffered saline, and the fluid removed from the lungs by vacuum. The lavage was centrifuged at 200 xg for 10 minutes and the supernatant recovered and centrifuged at 8-9000 xg for 20 minutes. The (surfactant) pellet was then suspended in 0.8M sucrose, which has a density greater than the buoyant density of the surfactant, and centrifuged at about 100,000 xg for three hours. The floating surfactant was then suspended in water and sedimented at about 9-10,000 xg for 20 minutes to remove the sucrose.

The phospholipid-rich surfactant was first extracted with 98% n-butanol, into which up to 2% aqueous surfactant (by volume) was added. This one-phase extraction allows solubilization of the 5 kd and 18 kd proteins and lipids while causing precipitation of the other proteins, which were removed by centrifuging at 9-10,000 xg. The butanol solution was then chromatographed over an LH-20 gel permeation column (Pharmacia) to separate the lipids from the 5 kd and 18 kd proteins. The desired protein peak was then rechromatographed over LH-60 which separates the 18 kd from the 5 kd protein. Both columns are run using chloroform: methanol (2:1, v:v) containing 0.5% 0.1N HCl.

The purified 5 kd and/or 18 kd proteins, either alone or in combination (1:1), were mixed in various weight ratios with synthetic phospholipids to obtain an effective surfactant.

### D.2. cDNA Encoding Canine 10K ASP Proteins

Messenger RNA extracted from adult canine lung tissue was used to prepare a DNA library using GC tailing in pBR322 as described in WO86/03408 (supra).

The SP-18 Protein: Two oligomeric probes were synthesized corresponding to the N-terminal sequence of the 18 kd protein using mammalian codon preference tables for codon choice. Probe 1198 was a 36-mer of the sequence 5'-GGTCACAGCCAGGCCCTTGGGGATCATGGCCTGGAT-3'; probe 1199 was a 45-mer of the sequence 5'-CTTGATCAGGGTTCTGCACAGCCAGCAGTAGGGCAGGGGGATGGG-3'. Both were labelled with ³²P by kinasing.

For hybridization, filters were baked at 80°C for two hours under vacuum and then washed for 4 hr at 68°C with shaking in a large volume of 3 x SSC containing 0.1% SDS. The filters were prehybridized for several hours at 42°C in 6 x SSC, 5 x Denhardt's, 20% formamide, 0.1% SDS, and 100 »g/ml sheared, denatured salmon sperm DNA. Duplicate filters were hybridized in the above buffer containing either 13 ng/ml probe 1198 or 16 ng/ml probe 1199 at an initial temperature of 68°C, and then at 42°C overnight. The filters were washed twice for 15 min at room temperature in 6 x SSC, 0.1% SDS, 0.05% sodium pyrophosphate, then for 5 min at 65°C in the same buffer, and then dried and autoradiographed.

Of 40,000 clones screened, 8 hybridized to both probes, and were subjected to restriction analysis. Two overlapping clones which when combined span 1520 nucleotides were sequenced, with the results shown in Figure 1. These two clones are designated pD10k-1 and pD10k-4, and are identified in Figure 1. The arrow indicates the beginning of the mature 18 kd protein.

cDNA encoding the SP-5 proteins: An oligomeric probe was synthesized which corresponded to the putative sequence of human 5 kd lung surfactant protein. A dog lung cDNA library was constructed as described above and screened. The cDNA isolated was approximately 800 bp. This was not a full-length cDNA, as Northern analysis showed that the full-length clone should be about 1.1 kb. The cDNA clone started approximately 30 amino acid residues upstream of the N-terminus of the mature dog 5 or 8 kd protein. A possible clip site (Gln-Gln) which would give a protein of approximately 5 kd.

### D.3. Human ASP DNAs

A human genomic library cloned into bacteriophage Charon 28 (Rimm, D. L., et al, Gene (1980) 12:301-310) was obtained from Dr. T. Maniatis, Harvard University. Approximately 1.5 x 10⁶ phage were grown on E. coli K803, and plaque lysates were transferred to nitrocellulose filters as described by Benton, W. D., et al, Science (1977) 196:180-182. Isolation of the genomic clone gHS-15 which encodes the 32 kd human protein and expression of this gene have already been described.

In addition, cDNA libraries from human lung were prepared as described previously either by GC tailing or in λgt10. The recovery of cDNA encoding the 32 kd human ASP protein was also described in WO86/03408.

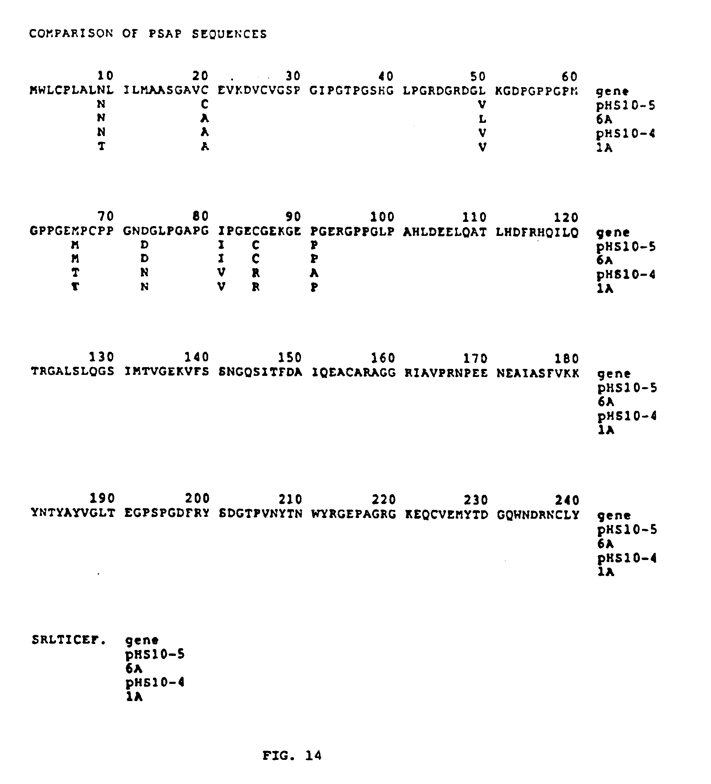

Disclosed herein, in Figure 14, are amino acid sequences encoded by cDNA clones obtained herein from the human lung library in λgt10 and designated pHS10-5 and pHs-10-4. These proteins differ by one and seven amino acids, respectively, from the protein encoded by the recovered genomic clone described in WO86/03408, which protein sequence is also shown in Figure 14. The remaining sequences of Figure 14, labeled 6A and 1A, are additional variants encoded by cDNAs obtained by others. It is believed that the 32K human ASP protein may be encoded by multiple genes.

Recovery of SP-18: As described in the published application, the cDNA library in λgt10 was screened on nitrocellulose filters using 1x10⁶ cpm of the canine clone pD10k-1 described above (and identified in Figure 1) in 40% formamide, 5 x SSC, 0.05% SDS, 5 x Denhardt's, 50 »g/ml yeast tRNA and 50 »g/ml salmon sperm DNA for 16 hr at 37°C. (The pD10k-4 segment or the full-length combination of the pD10k-1 and pD10k-4 clones can be used as well.) The filters were washed twice at 50°C for 30 min in 2 x SSC, 0.1% SDS, dried and autoradiographed. Of 40,000 plaques, two were positive, and one, designated cDNA 3 containing a 1.5 kb insert was chosen for sequencing. The complete nucleotide and deduced amino acid sequence for the SP18 protein and its precursor are shown in Figure 2. The mature SP18 protein begins, as shown in the Figure, at nucleotide 614 with the Phe at 201. It is believed that the carboxy terminus of the processed protein is the arginine at position 286. The 1.5 kb insert was excised and subcloned into EcoR1-cut pUC8; this plasmid, designated as ph18K-3, was deposited in E. coli K-12 strain MC1061 with the American Type Culture Collection under ATCC accession no. 67276.

The ph18K-3 cDNA insert was used to screen the human genomic library (supra) for the gene encoding the SP18 protein and its precursors. The sequences of the coding exons of the recovered gene are shown in Figure 3. The mature amino terminus at Phe-201, is at nucleotide 3866; the numbering of the genomic nucleotide sequence begins with the first residue of the 7332 bp that were sequenced from the lambda clone.

The genomic and cDNA coding sequences differ at a single nucleotide, resulting in amino acid sequences for the precursor that differ by a single residue; Ile-131 of the cDNA appears as Thr-131 in the genomic clone. Thus, the genomic clone-encoded precursor contains two consensus sites for N-linked glyosylation (Asn-129:Thr-131 and Asn-311:Ser-313), the cDNA-encoded sequence contains only the latter glycosylation site. It is expected that cDNA clones encoding the genomic sequence are also present in the library.

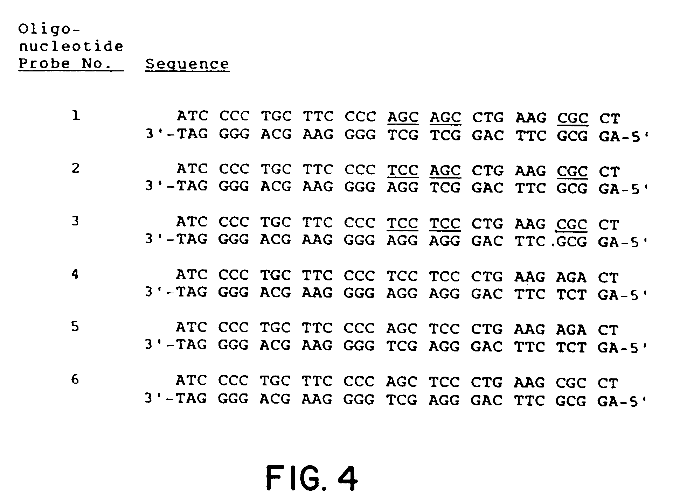

Recovery of SP-5: For the SP5 proteins, a nucleotide mixture of 6 oligonucleotides was pooled (Figure 4), which nucleotides were made to the N-terminal amino acid sequence of dog 8 kd and 5 kd protein. The human lung cDNA library in λgt10, prepared as described above, was screened, and 8 cDNAs encoding the SP5 protein were obtained. A cDNA clone starting approximately 19 residues upstream from the putative N-terminus of the mature SP-5 protein contains 820 bp and was inserted in lambda phage, designated λh6K-3, and deposited with the American Type Culture Collection under ATCC accession no. 40294.

Two representative cDNA clones, Nos. 18 and 19 are shown in Figures 5 and 6. cDNA # 18 contains the longest insert, of 862 bp, including 12 residues of poly(A); however, from Northern blot analysis, the mRNA encoding the SP-5 protein is 1-1.1 kb in length. cDNAs #s 18 and 19 differ by 4 nucleotides, underlined in the cDNA # 19 sequence, which result in two amino acid differences: Asn-138 in # 18 is Thr-138 in # 19, and Asn-186 in # 18 is Ser-186 in # 19.

There are two N-terminal amino acid residues seen in the human 5 kd and 8 kd proteins, corresponding to Phe-24 and Gly-25 in Figures 5 and 6. The carboxy termini of the 5 kd and 8 kd proteins have not been precisely determined; it is postulated that the 8 kd protein ends at Gln-108, while the 5 kd protein ends at Glu-80 or at Thr-65.

(A canine lung library in pBR322 was prepared substantially as described above and screened with the human 820 bp clone. The isolated cDNA -- designated pD6k-11 -- was about 800 bp (see Figure 13), not a full-length cDNA. The clone started approximately 30 amino acid residues upstream of the N-terminus of the mature canine SP-5 protein, and contained a possible Gln-Gln clip site.)

### D.4. Construction of Mammalian Expression Vectors

Vectors suitable for expression of the various ASP encoding sequences in mammalian cells, which are also capable of processing intron-containing DNA were constructed. Expression is controlled by the metallothionein II (hMTII) control sequences, as described by Karin, M., et al, Nature (1982) 299:797-802.

An intermediate host vector, pMT was obtained by ligating the promoter into pUC8 as follows:
Plasmid 84H (Karin, M., et al (supra)) which carries the hMTII gene was digested to completion with BamHI, treated with exonuclease Bal-31 to remove terminal nucleotides, and then digested with HindIII to liberate an 840 bp fragment containing nucleotides -765 to +70 of the hMTII gene (nucleotide +1 is the first nucleotide transcribed). The 840 bp fragment was isolated and ligated with HindIII/HincIl digested pUC8 (Vieira, J., et al. Gene (1982) 19:259-260) and the ligation mixture transformed into E. coli MC1061. The correct construction of pMT was confirmed by dideoxy nucleotide sequencing.

In addition, a derivative of the pMT, pMT-Apo, containing C-terminal regulatory signals was also prepared. pMT-Apo harbors a portion of the human liver protein apoAI gene (Shoulders, C. C., et al, Nucleic Acids Res (1983) 11:2827-2837) which contains the 3'-terminal regulatory signals. A PstI/PstI 2.2 kb fragment of apoAI gene (blunt ended) was cloned into the SmaI site of the pMT polylinker region, and the majority of the apoAI gene removed by digestion with BamHI, blunt ending with Klenow, digestion with StuI, and religation. The resulting vector contains roughly 500 bp of the apoAI gene from the 3' terminus as confirmed by dideoxy-sequence analysis.

Additional expression vectors containing the SV40 viral enhancer were also constructed by insertion of an 1100 bp SV40 DNA fragment into the HindIII site preceding the MT-II promoter sequences in pMT. The SV40 DNA fragment spans the SV40 origin of replication and includes nucleotide 5171 through nucleotide 5243 (at the origin), the duplicated 72 bp repeat from nucleotide 107-250, and continues through nucleotide 1046 on the side of the origin containing the 5' end of late viral mRNAs. This HindIII 1100 bp fragment is obtained from a HindIII digest of SV40 DNA (Buchman, A.R., et al, DNA Tumor Viruses, 2d ed (J. Tooze, ed.), Cold Spring Harbor Laboratory, New York (1981), pp. 799-841), and cloned into pBR322 for amplification. The cloning vector was cut with HindIII, and the 1100 bp SV40 DNA fragment isolated by gel electrophoresis and ligated into HindIII-digested, CIP-treated, pMT. The resulting vectors, designated pMT-SV(9) and pMT-SV(10), contain the fragment in opposite orientations preceding the MT-II promoter. In pMT-SV(9), the enhancer is about 1600 bp from the 5' mRNA start site; in the opposite orientation SV(10) it is approximately 980 bp from the 5' mRNA start site. Both orientations are operable, but the orientation wherein the enhancer sequences are proximal to the start site provides higher levels of expression.

The 500 bp apoAI fragment was inserted into pMT-SV(10) by isolating this fragment, obtained by digestion of pMT-Apo (described above) and ligating the isolate into EcoRI/BamHI digested pMT-SV(10) to obtain the desired host vector: pMTApo10.

This host vector was digested with BamHI, blunted, and ligated to the cDNA sequences obtained from the clone # 3 of 1275 bp encoding SP-18 precursor, shown in Figure 2 as a blunted fragment. This was done by isolating an EcoRI/BamHI (partial) fragment from cDNA #3 (Figure 2) avoiding the BamHI site at nucleotide 663, and subcloning into EcoTI/BamHI pUC9 the desired fragment was excised with EcoRI and HindIII, blunted with Klenow, and then inserted into pMTApo10. The resulting vector, pMT(E):SP18-40k, was transformed into CHO cells as described below.

In a similar manner, the blunted EcoRI insert of the SP-5 clones of Figures 5 and 6 was placed into BamHI digested pMTApo10 to obtain pMT(E):SP-5 vectors, and transformed into CHO cells.

### D.5. Expression in Mammalian Cells

Chinese hamster ovary (CHO)-K1 cells were grown on medium composed of a 1:1 mixture of Coon's F12 medium and DME21 medium with 10% fetal calf serum. The competent cells were co-transformed with the vector of interest and pSV2:NEO (Southern, P., et al, J Mol Appl Genet (1982) 1:327-341). pSV2:NEO contains a functional gene conferring resistance to the neomycin analog G418. In a typical transformation, 0.5 »g of pSV2-NEO and 5 »g or more of the expression vector DNA were applied to a 100 mm dish of cells. The calcium phosphate-DNA co-precipitation according to the protocol of Wigler, M., et al, Cell (1979) 16:777-785, was used with the inclusion of a two minute "shock" with 15% glycerol in PBS after four hours of exposure to the DNA.

Briefly, the cells are seeded at 1/10 confluence, grown overnight, washed 2x with PBS, and placed in 0.5 ml Hepes-buffered saline containing the CaPO₄·DNA co-precipitate for 15 min and then fed with 10 ml medium. The medium is removed by aspiration and replaced with 15% glycerol in PBS for 1.5-3 min. The shocked cells are washed and fed with culture medium. Until induction of MT-II-controlled expression, the medium contains F12/DMEM21 1:1 with 10% FBS. A day later, the cells are subjected to 1 mg/ml G418 to provide a pool of G418-resistant colonies. Successful transformants, also having a stable inheritance of the desired plasmid, are then plated at low density for purification of clonal isolates.

The transformants are assayed for production of the desired protein, first as pools, and then as isolated clones in multi-well plates. The plate assay levels are somewhat dependent on the well size - e.g. results from 24 well plates are not directly comparable with those from 96 well plates. Clones which are found by plate assay to be producing the protein at a satisfactory level can then be grown in production runs in roller bottles. Typically, the levels of production are higher when the scale up is done. However, there is not an absolute correlation between performance in the plate assay and in roller bottles - i.e. cultures which are the best producers in the plate assay are not necessarily the best after scale-up. For this reason, typically 100-200 or more individual clones are assayed by various screening methods on plates and 5-10 of the highest producers are assayed under production conditions (roller bottle).

Pools of transformed cells were grown in multi-well plates and then exposed to 5 x 10⁻⁵ to 1 x 10⁻⁴ zinc ion con- centration to induce production of ASP.

Semiconfluent monolayers of individual cell lines growing in McCoy's 5A medium with 10% FBS were washed with phosphate-buffered saline (PBS) and refed with McCoy's containing 10% FBS, 1 x 10⁻⁴ zinc chloride, and 0.25 mM sodium ascorbate. (Ascorbate may be helpful in mediating the hydroxylation of proline residues.) Twenty-four hours post induction, the cells were washed with PBS and refed with serum-free McCoy's containing the zinc chloride and ascorbate. After 12 hours, the conditioned media were harvested.

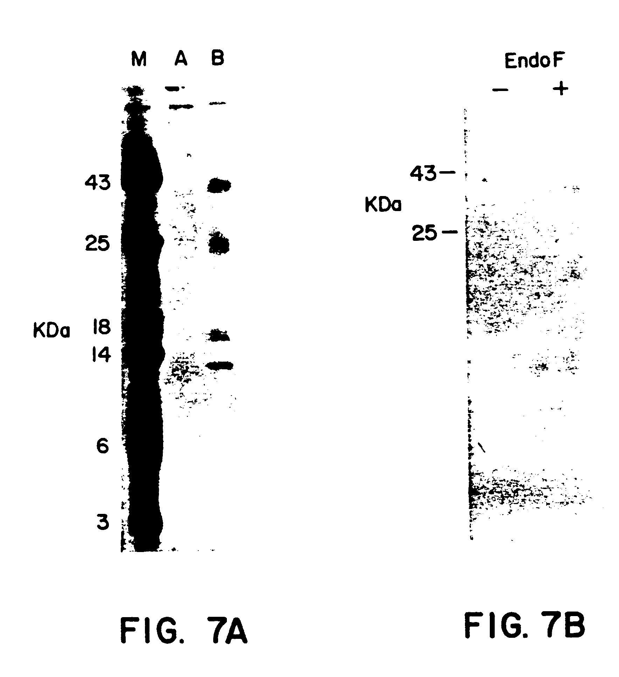

A pool of transformed cells was induced with ZnCl₂ as described above, and labeled with ³⁵S-methionine. After a 12 h labeling period, culture medium was harvested as described and immunoprecipitated with antisera raised against the human SP-18 ASP. Samples were then subjected to SDS PAGE in a 15% gel, with the results shown in Figures 7a and 7b.

In Figure 7a, lane M represents molecular weight standards, lane A represents immunoprecipitated proteins from untransformed CHO cells, and lane B represents immunoprecipitated protein from the pMT(E):SP18-40k transformed pool. In Figure 7b, the immunoprecipitated protein from transformed ppol was digested with endoglycosidase F for one hour, then electrophoresed as in Figure 7a. Lane A is untreated control, lane B is the digested sample.

As shown in Figure 7a, 43 kd and 25 kd precursor proteins are produced by the transformed cells; the smaller molecular weight proteins shown in Figure 7a are not reproducible. The results of Figure 7b show the 43 kd precursor is glycosylated. The size of the unglycosylated, immunoprecipitated protein is that predicted for the full-size precursor.

Cold protein produced by the above induced pool was subjected to Western blot using antisera raised against a peptide spanning residues 336-353 of the precursor. It is believed the 25 kd product represents a 181 amino acid sequence spanning Phe 201-Leu-381, containing a N-linked glycosylation site.

### D.6. Additional Vectors

Analogous vectors were constructed using standard site-specific mutagenesis techniques to provide sites for in vitro cleavage of the precursor protein which was, apparently produced in CHO cells from the full length sequence. In one such construct, the 381 amino acid precursor was modified to replace each of Gln-199:Gln-200 and Arg-286:Ser-287 by Asn:Gly, to provide sites cleavable by hydroxylamine (which cleaves between Asn and Gly). Cleavage of the precursor thus produced with hydroxylamine generates the putative mature form, with an additional Gly residue at the amino terminus, and with the putative carboxy-terminal Arg-286 changed to an Asn residue.

In another construct. Phe-201 and Ser-287 are changed to ASP residues. Cleavage with acid (between Asp and Pro) yields a mature form of the SP-18 protein missing the N-terminal Phe-201, and with an additional carboxy-terminal Asp residue.

An additional construct allows in vitro processing of the precursor with a more gentle, enzymatic procedure, employing Staph V8 peptidase, which cleaves after Glu residues. Advantage is taken of natural Glu residues at Glu-198 and Glu-291 by converting the Glu-251 to Asp. The 43 kd precursor is cleaved with Staph V8 to yield the putative mature SP-18 protein with an additional Gln-Gln at the amino terminus, and Pro-Thr-Gly-Glu at the carboxy terminus. In an additional construct, Glu residues can be placed in positions 200 and/or 287.

### D.7. Expression in Bacteria

The unglycosylated form of the SP-18 protein can be produced in bacteria as a 181 amino acid precursor representing met-preceded residues 201-381 or as a hydroxylamine-cleavable fusion protein precursor with a 15 residue β-galactosidase leader. A modified cDNA encoding amino acids 201-381 of the cDNA, preceded by ATG is inserted into the Trp controlled vector, pTrp-233 (pTrp host vector), between the EcoRI site and the HindIII site to give pTrp-20. This construct produces a protein of M.W. 20 kd. An analogous construct in pBGal host vector, pBGal-20 contains the same sequences of SP18 cDNA # 3 fused to a 15 residue β-galactosidase leader through a hydroxylamine-sensitive Asn-Gly doublet, and produces a fusion protein of MW = 22 kd. Details of the construction are given in D.11. below.

The pTrp-20k and pBGal-20k plasmids were used to transform E. coli W3110 to ampicillin resistance. Rapidly growing cultures of pTrp-20/W3110 or pBgal-20/W3110 in M9 medium (1 x M9 salts, 0.4% glucose, 2 mg/ml thiamine, 200 »g/ml MgSO₄.7H₂O, 0.5% casamino acids, 100 »g/ml IAA (3-β indoleacrylate, Sigma I-1625) to induce the trp promoter.

The induced cells were allowed to grow for 2 hours before labeling with ³⁵S methionine (100 uCi/ml cells) for 10 minutes. The labeling was stopped by the addition of 350 »l cold 20% TCA per ml of cells; the TCA pellets were washed with acetone, and then resuspended by boiling in SDS PAGE sample buffer, and subjected to PAGE in a 15% gel.

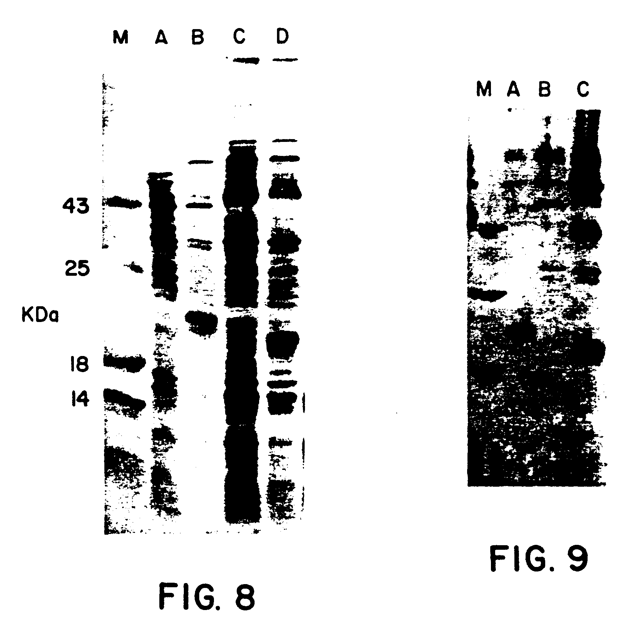

Figure 8 shows the results of this procedure: lane M is size standards; lane A is pBgal host vector/W3110, lane B is Bgal-20/W3110, lane C is pTrp host vector/W3110, and lane D is pTrp-20/W3110. Lanes B and D show major labeled proteins of 22 kd and 20 kd, respectively, which are not present in lanes A and C.

Cold extracts of the induced cells were prepared the same way, subjected to PAGE, then Western blotted to nitrocellulose, using antisera raised against a peptide corresponding to amino acids 336-353, and then with ¹²⁵I-Protein A. In Figure 9, lane A is Bgal-20/W3110, lane B is pTrp host vector/W3110, and Lane C is pTrp-20/W3110. It is clear that both pTrp-20 and Bgal-20 show immunospecific proteins of the predicted molecular weight.

Vectors encoding modified SP-18 protein sequences providing cleavage sites as set forth above for expression in bacteria were also prepared as follows. In pTrp-20, codons encoding Arg-286 Ser-287 were altered to encode Asn-Gly; introducing the hydroxylamine-sensitive cleavage site, or the codon for Ser-287 was replaced by a codon for Asp, resulting in the acid-sensitive Asp-Pro cleavage site; or the codon for Glu-251 was replaced with a codon for Asp, allowing cleavage with Staph V8 at Glu-291 without cleaving the desired protein. Also, in both pTrp-20 and pBGal-20, the sequences 3' to the putative carboxy terminal Arg-286 were deleted and replaced by a stop codon. Neither construct resulted in labeled protein of proper size after induction.

Analogous to pTrp-20, the desired fragment of the cDNA # 18 (Figure 5) extending from Gly-25 preceded by ATG to the carboxy-terminal Ile-197 of the SP-5 "precursor" was inserted into EcoRI/HindIII digested pTrp-233 to give pTrp-5 and into pBGal host vector to give pBGal-5 wherein the SP-5 sequence is fused to a β-galactosidase leader through a hydroxylamine-sensitive Asn-Gly.

Also, cleavage with Staph V8 of the protein expected from this construct at the Glu preceding Phe-24 and at Glu-66 yields mature 5 kd protein if the putative C-terminus is correct.

These constructs are transformed into E. coli W3110 and expressed as described above.

### D.8. Activity of the ASP Components

The ability of the isolated ASP components to enhance the formation of lipid film at an air/aqueous interface was assessed in vitro using the method described by Hagwood, S., et al, Biochemistry (1985) 24:184-190. Briefly, a preparation of phospholipid vesicles with the appropriate ratio of test proteins is added carefully in a small volume to the bottom of a teflon dish containing aqueous buffer, a magnetic stirrer, and a platinum plate suspended at the surface of the buffer and attached to a strain gauge. Changes in surface tension registered on the strain gauge are recorded as a function of time upon starting the stirrer.

10K proteins were added to the phospholipid by mixing a chloroform solution containing them with a 2:1 v/v chloroform:methanol solution of the lipid. The solvents were evaporated, and the solids hydrated in buffer to obtain vesicles. 32K proteins can be added in aqueous solution directly to a suspension of the vesicles, and association with and aggregation of the vesicles can be detected by turbidity measurements.

As reported by Hawgood, et al (supra), 32K canine ASP was capable of aggregating phospholipid vesicles and of enhancing the formation of film when included in the phospholipid vesicles, when the phospholipids were those obtained from the canine lung surfactant complex. The activity of the proteins of the invention is assessed using the same procedures for measuring aggregation and film formation enhancement as set forth in Hawgood.

Both the phospholipid preparation from canine lung prepared as described above (300 »g) and a synthetic mixture of phospholipids were used. The synthetic phospholipid contained 240 »g of commercially available DPPC and 60 »g egg PG, and is much more reluctant to form films than is the natural lipid. However, the test phospholipid was chosen so as to dramatize most effectively the activity of the proteins.

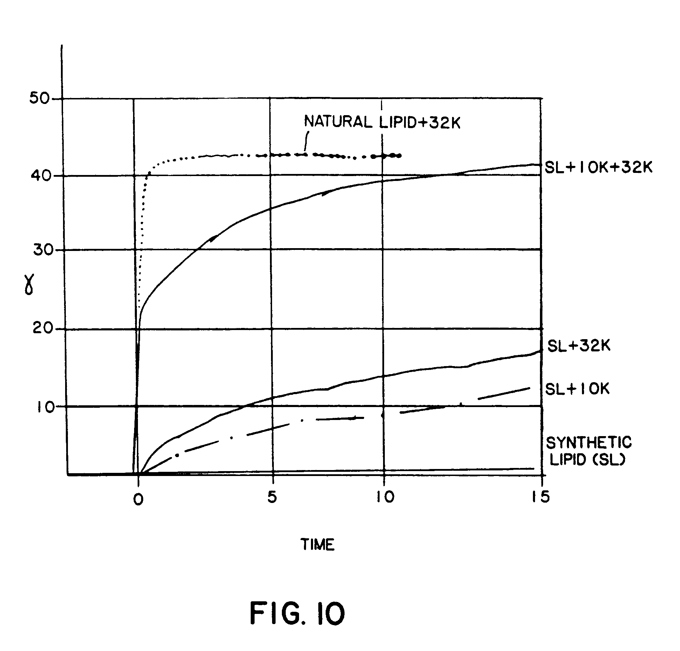

The 32K protein and the mixture of 10K ASP were isolated from canine lung as described above. While the addition of 60 »g of the 32K protein was able to enhance film formation by the "natural" phospholipid obtained from lung almost to the level exhibited by the complex per se, it only moderately enhanced film formation using synthetic lipid. Similar results were obtained for addition of 13 »g of the 10K protein alone. However, when 13 »g of the 10K preparation was incubated with the synthetic phospholipid vesicles prior to the addition of 60 »g of 32K protein, film formation occurred at a rate and to a degree comparable to that of the natural complex per se. These results are shown in Figure 10.

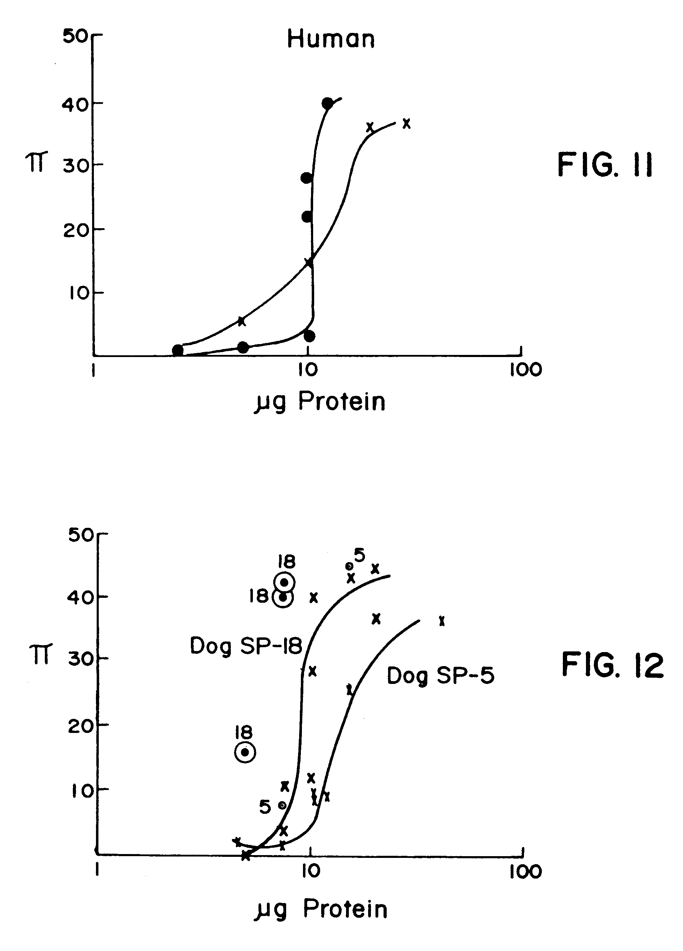

The results for individual human and canine 5 kd and 18k proteins are shown in Figures 11 and 12, plotting surface pressure after 3 minutes (y axis) versus protein concentration (x axis). As shown in Figure 11, the maximum pressure attained is 40-45 mN/m, and either 5 kd or 18k cause the spreading of lipids at about 10 »gs this corresponds to a phospholipid-to-protein ratio of 10:1 since 100 »g of lipid was used in all cases; the lipid mixture was DPPC:PG (7:3), but 8:2 and 9:1 ratios gave no significant difference in results.

For the canine proteins shown in Figure 12, the results are identical to those for the human protein. Figure 12 also shows the results of experiments in which recombinantly produced r32K was added to the 18 kd or 5 kd protein. The synergy between the proteins is shown in the circled dots. Ten »g 32 kd protein was added to 5 »g and 7.5 »g for 18 kd, and 7.5 and 11 »g for 5 kd protein.

Bovine 18 kd and 5 kd proteins gave identical results to the canine and human proteins.

### D.10. In Vivo Tests

The control surface active material (SAM) for in vivo testing was prepared as follows. Lungs of young adult rabbits are lavaged with saline. Healthy rabbits are anesthetized through the ear vein with 3 cc of sodium pentobarbital. The trachea is exposed and a 3-way stopcock with a tube attached is inserted into the trachea and secured. The chest is opened, the chest walls are removed, and the pulmonary artery is catheterized with a size 8 feeding tube from the heart. The circulation is flushed with 50 ml of normal saline while ventilating the lungs through the tracheal tube with a 60-ml syringe, and the lungs are then carefully removed with the trachea intact. Sixty ml of normal saline are instilled into the lungs through the tracheal tube, the lungs are then gently massaged for one minute, and the saline is withdrawn. Lavage is repeated four times, and the washings are pooled. Cell debris is removed from the lavage fluid at room temperature by centrifugation at 1000 x g for two hours. The pellet is suspended in 0.1 N saline plus 2 M CaCl₂ at a final concentration of 10 mg/ml phospholipid. Concentration is adjusted by extracting the lipids with choloroform and methanol and measuring lipid phosphorus. In the bubble tensitometer this material give rapid adsorption (time constant 0.3 sec or less) and minimum surface tensions of 0 to 3 mN/m on 50% reduction of area. Maximum tension on expansion was 32 to 35 mN/m.

The subject and apparatus used for in vivo testing are as follows. Healthy, young, time-dated pregnant does are obtained from White Hare Rabbitory of Missouri. At 21 or 22 days gestation the does are air-shipped and are checked upon arrival to assure that they are pregnant and healthy. Does are housed in standard large rabbit cages in the rabbit facility (1492-S) and are re-examined the day before use.

Four plethysmographs were constructed with 80inch lengths of 2-inch diameter acrylic cylinder to which are affixed a 3-inch long chimney of 1/2-inch acrylic tubing (id, 0.5 inch). The chimney is filled with enough cotton gauze to create a low resistance to air flow in and our of the plethysmograph. Flow in and out of the chamber is determined by measuring the differential pressure change between the inside of the plethysmograph and the room. (Time constant <0.1 seconds). Leads are taken from the end of the main cylinder to a pressure transducer (Validyne DP45, Validyne Engineering Company, Northridge, CA) and to a calibrating syringe. When conducting experiments, the electrically integrated flow (volume) signal is frequently calibrated with the syringe. The other end of the main cylinder is sealed with a 2-inch rubber stopper through which were placed two 4-8nch metal rods and through which were pulled three ECG leads. Cotton sheeting is placed between the two metal rods forming a sling on which the experimental animal is placed. Bayonet-type electrodes are attached to the ECG leads. aN adapter is placed through the stopper so that the hub of the tracheal angiocath can be connected to a flow-through manifold which in turn is attached to the tubing from a respirator (MarK VIII, Bird Respirator Company, Palm Springs, CA). The external deadspace of the airway is 0.05 to 0.07 ml. Airway pressure is measured in the manifold with an Alltech MSDICE/1 transducer (Alltech, City of Industry, CA). The plethysmograph calibration is linear at volumes of 0.01 to 1 ml and at frequencies of 10 to 100 oscillations per minute. The four plethysmographs are mounted in a single water bath heated to 37°C. each animal has it own ventilator. Switching devices permit flow, volume, airway pressures and ECG to be recorded from each rabbit sequentially on a Brush recorder. Usually three animals are used for one minute in every five minutes from each is recorded.

The procedure used was as follows. Rabbit pups of 27 d ± 4 hr gestation were used. After giving the dose spinal anesthesia (1 ml pontocaine), the abdomen is opened and the uterus exposed. Two minutes before opening the uterus, each fetus receives 15 mg/kg pentobarbital and 0.1 mg/kg pancuronium intraperitoneally. When fetal movement stops, the fetuses are anesthetized and quickly delivered. After weighing, three pups of about the same weight are chosen for the experiment. Pups with obvious anomalies are not studied. Pups must be between 22 and 40 grams weight (mean ± 2 SD). Tracheas are cannulated with 18-gauge angiocaths while they are kept warm under radiant heat. After cannulation, 0.2 ml of either saline, SAM or test substance (warmed to 37°C in H₂O bath and then passed through a 25 g needle x 5 to insure uniform mixing of the material) are put into the trachea of the three matched pups from each litter while gently squeezing the chest until lung fluid appears at the needle hub in order to create a fluid-to-fluid interface. The treatment is followed by 0.45 ml of air.

All test substances (but not saline or SAM controls) contain 50 mg phospholipid/kg delivered as 0.2 ml per animal at 10 mg phospholipid/ml. Concentration and dose are constants for each study. The animals are placed on the slings, and the ECG electrodes attached and the tracheotomy tube connected to on adapter connected to a respirator. The average elapsed time from delivery to the beginning of assisted ventilation is 10 minutes, maximum elapsed time is 15 minutes. Ventilation is begun with oxygen at a frequency of 48 breaths/minute using an inspiratory time of 0.35 seconds. For the first minute, the ventilatory settings are the same for all animals; inspiratory time 0.35 seconds, peak inspiratory pressure 40 cmH₂O. After the first minute the inspiratory pressure is adjusted to keep the tidal volume at 6.5 - 7.5 ml/kg. Animal weight is about 30 g so this is achieved with an absolute volume of about Ol21 ml. The flow, tidal volume and airway pressure are recorded every five minutes for each of the three littermates. Animals are ventilated for 30 minutes.

Data from all animals in a set are rejected if one member develops an air leak or dies of other causes.

After 30 minutes ventilation the tracheal tubes are closed with stopcocks and the lungs are allowed to degas for 10 minutes. Then each air-filled angiocath is connected to a horizontal, calibrated length of 5 mm plastic tubing containing 3 ml of air at the lung end and dyed water at the other end. The fluid-filled ends of three plastic tubes are connected via a manifold to a single reservoir of dyed water, whose surface is at the same level as the tubes. This reservoir can be raised in 50 cm water steps which correspondingly increase the pressure in the tubing and lungs. As the pressure increases or decreases, gas enters or leaves the lungs, displacing the fluid column, allowing measurement of the changes in gas volumes. This apparatus is similar to that described by Robertson, B. Lung (1980) 158:57-68. The pressure is raised stepwise from 0 to 5, 10, 15, 20, 25, 30 cmH₂O, with a pause for one minute at each level before recording the volume change. after one minute at 20 mmH₂O, the pressure is decreased by 5 cmH₂O decrements, again maintaining each pressure for one minute before recording the volume. Each volume measurement is corrected for compression. During the studies the animals are kept at 37°C by placing them in a water bath just below the surface.

Data are obtained for P_{INS}, compliance (C) and volume at specific pressures (Vp). P_{INS} is the pressure required to maintain a net lung volume; lower numbers, of course, indicate efficacy. Compliance, a measure of how easily the lungs are inflated, is also measured, and higher values are desired. Vp is the volume in cm³ of the lungs at the noted pressure in cm of water. The results are as follows.

For P_{INS} at 30 minutes, the results are as in Table 1 (PL is phospholipid; 32K protein is human 32 kd ASP produced in CHO cells; 10K is a mixture of 5 kd, 8 kd and 18 kd isolated native human proteins).

**Table 1**

| TREATMENT | n | P_{INS} |
|---|---|---|
| SAM | 13 | 18 ± 4 |
| Saline (control) | 9 | 31 ± 1 |
| PL alone | 4 | 32 ± 1 |
| PL + 32K | 3 | 28 ± 5 |
| PL + 10K | 8 | 17 ± 2 |
| PL + 10K (200:1) + 32K (4:1) | 8 | 20 ± 5 |
| PL + 10K (200:1) | 5 | 25 ± 8 |
| PL + 18 kd (50:1) | | 21, 22, 20 |
| PL + 5 kd (50:1) | | 19 |

As shown in Table 1, 32K alone is minimally effective, while the 10K mix or 5 kd or 18 kd proteins alone are reasonably effective. Addition of the 32K protein to the 10K mix, however, enhances the effectiveness synergistically.

For compliance, Table 2 shows similar results.

**Table 2**

| TREATMENT | n | Compliance |
|---|---|---|
| SAM | 13 | 0.441 ± 0.113 |
| Saline (control) | 9 | 0.243 ± 0.025 |
| PL alone | 4 | 0.219 ± 0.028 |
| PL + 32K | 3 | 0.247 ± 0.029 |
| PL + 10K | 8 | 0.467 ± 0.078 |
| PL + 10K (200:1) + 32K (4:1) | 8 | 0.401 ± 0.041 |
| PL + 10K (200:1) | 5 | 0.328 ± 0.176 |
| PL + SP-18 (50:1) | | 0.4 ± 0.045 |
| PL + 5 kd (50:1) | | 0.4 ± 0.045 |

Again the 10K mix or the 18 kd and 5 kd proteins show good activity, and while the 32K is much less effective, addition of the 32K protein greatly enhances activity of the 10K mix.

Tables 3 and 4 show V₃₀ and V₅, (30 cm water and 5 cm water) respectively.

**Table 3**

| TREATMENT | n | V₃₀ |
|---|---|---|
| SAM | 12 | 72 ± 9 |
| Saline (control) | 4 | 23 ± 11 |
| PL alone | 1 | 26 |
| PL + 32K | 3 | 38 ± 15 |
| PL + 10K | 7 | 65 ± 9 |
| PL + 10K (200:1) + 32K (4:1) | 7 | 58 ± 11 |
| PL + 10K (200:1) | 2 | 55 ± 33 |

**Table 4**

| TREATMENT | n | V₅ |
|---|---|---|
| SAM | 12 | 56 ± 9 |
| Saline (control) | 4 | 11 ± 7 |
| PL alone | 2 | 14 |
| PL + 32K | 3 | 20 ± 7 |
| PL + 10K | 7 | 48 ± 9 |
| PL + 10K (200:1) + 32K (4:1) | 7 | 45 ± 10 |
| PL + 10K (200:1) | 2 | 43 ± 27 |

The results track those obtained for P_{INS} and compliance in the preceding tables.

Results obtained with the corresponding bovine proteins are similar.

### D.10. Host Vectors

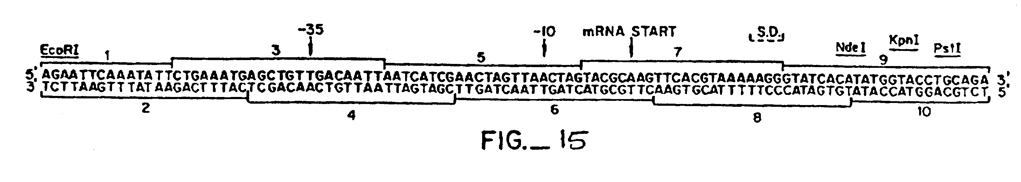

pTrp233 is prepared from pKK233-2, which is described in detail in Amann, E., et al, Gene (1985) 40:183-190, by replacing the tac promoter of pKK233-2 with a synthetic trp promoter of the nucleotide sequence shown in Figure 15. The NdeI site of the starting plasmid is eliminated by digesting pKK233-2 with NdeI, blunting with Klenow, and religating. The NdeI-minus product was then digested with EcoRI and PstI, and ligated to an EcoRI/PstI digest of the synthetic trp promoter of Figure 15 to obtain the desired vector pTrp233. To prepare pBGal host vector, pTrp 233 was digested with EcoRI, purified on a gel, and blunted with Klenow. The plasmid was relegated and amplified in E. coli to give the corresponding plasmid lacking the EcoRI site. A synthetic oligonucleotide sequence encoding the amino terminus of β-galactosidase followed by 6 threonino residues, 
beta-gal                    (thr)6
[1] TATGACCATGATTACGAATTTAACCACCACCACCACCACCGAATTCATTA     [3]
[2]   ACTGGTACTAATGCTTAAATTGGTGGTGGTGGTGGTGGCTTAAGTAATTCGA  [4]
    NdeI                                          EcoRI      HindIII
was ligated into NdeI/HindIII digested intermediate plasmid, and plasmids containing the insert (pBGal host vector) identified by susceptibility to EcoRI cleavage.

To construct pTrp-20, a portion of the SP-18 cDNA #3, along with a synthetic fragment, was ligated into NdeI/HindII digested pTrp-233. The SP-18 fragment ligated into pUC-9 described above was excised by digesting with PstI (cuts at nucleotide 694) and with HindIII (cuts past the 3' end in the plasmid polylinker). Two oligonucleotides were prepared, which, when annealed, encode the residues upstream of nucleotide 694 to the N-terminus (residue 201) and a preceding methionine (ATG): TATGTTCCCCATTCCTCTCCCCTATTGCTGGCTCTGCA and GAGCCAGCAATAGGGAGAGGAATGGGGAACA. These oligonucleotides were annealed, ligated to the excised cDNA, and inserted into the digested vector to obtain pTrp-20.

To construct pBGal-20, an analogous procedure using EcoRI/HindIII digested pBGal host vector, PstI/HindIII excised SP-18 DNA, and the filler nucleotides: AATTGAACGGTTTCCCCATTCCTCTCCCCTATTGCTGGCTCTGCA and GAGCCAGCAATAGGGGAGAGGAATGGGGAAACCGTTG, to give pBGal-20.

Vectors for the expression of the gene encoding shorter forms of SP-18 were constructed from pTrp-20 or pBGal-20. To construct pTrp-9, pTrp-20 was cut with Ncol (nucleotide 846) and HindIII, and rejoined with the annealed oligonucleotides CATGGATGACAGCGCTGGCCCAGGGTA and AGCTTACCTTGGGCCAGCGCTGTCATC. pBGal-9 was constructed in a completely analogous manner using pBGal-20 as starting material.

To construct vectors encoding SP-5, cDNA #18 (Figure 5) was digested with SmaI (nucleotide 94 - nucleotide 680) and the SmaI-excised fragment inserted into the SmaI site of pUC8. From the cloned gene, the fragment excised by digestion with ApaLI (nucleotide 123) and HindIII (linker) was ligated with NdeI/HindIII digested pTrp-233 and the joining annealed nucleotides: TATGGGCATTCCCTGCTGCCCAG and TGCACTGGGCAGCAGGGAATGCCCA, to obtains pTrp-5.

Similarly, pBGal-5 (N:G) and pBGal-5 (V8) were constructed using the same cDNA excised fragment, pBGal host vector cut with EcoRI and HindIII, and the nucleotide pairs: AATTCAACGGCATTCCCTGCTGCCCAG and TGCACTGGGCAGCAGGGAATCCCGTTG; and AATTCGGCATTCCCTGCTGCCCAG and TGCACTGGGCAGCAGGGAATGCCG, respectively.

## Claims (Claims for the following Contracting State(s): BE, CH, DE, FR, GB, IT, LI, LU, NL, SE)

1. An alveolar surfactant protein (ASP) free of proteins normally accompanying it in situ, which is
(a) isolated and purified human ASP having the amino acid sequence of the mature protein encoded by the DNA shown in Figure 2, having an amino terminus at position 201 of said figure or encoded by a naturally occurring allelic variant thereof;
(b) isolated and purified human ASP having the amino acid sequence of the mature protein encoded by the DNA shown in Figure 5 or Figure 6, and having a molecular weight of approximately 5 kd, or encoded by a naturally occurring allelic variant thereof; or
(c) isolated and purified canine ASP having the amino acid sequence of the protein encoded by the DNA shown in Figure 13, having a molecular weight of approximately 5 kd, or encoded by a naturally occurring allelic variant thereof.

2. A protein according to claim 1 which is encoded by the DNA sequence shown in Figure 2 (having an amino terminus at position 201 of said Figure), Figure 5, Figure 6 or Figure 13.

3. A protein according to claim 1 or 2 which is encoded by human or canine SP-5 DNA.

4. A protein according to claim 1, 2 or 3 which is encoded by human DNA.

5. A recombinant expression vector comprising a DNA sequence which encodes the protein claimed in any one of claims 1 to 4, which DNA is free of DNA encoding proteins normally accompanying the proteins of claim 1, operably linked to control sequences effective in expressing said sequence in suitable recombinant host cells.

6. Recombinant host cells transformed with the expression vector of claim 5.

7. A method of producing ASP which comprises culturing the cells of claim 6.

8. A pharmaceutical composition effective in treating respiratory distress syndrome (RDS) in mammals which composition comprises a protein according to any one of claims 1 to 4 in admixture with a phospholipid preparation and, optionally with a pharmaceutically acceptable excipient.

9. A composition according to claim 8 which further comprises an effective amount of 32K ASP protein.

10. A composition according to claim 8 or 9 which comprises from 50% to almost 100% by weight phospholipid.

11. A composition according to any one of claims 8, 9 or 10 which comprises dipalmitoyl phosphatidylcholine (DPPC).

## Claims (Claims for the following Contracting State(s): AT, ES, GR)

1. A process for producing recombinant host cells comprising transforming them with a recombinant DNA sequence which is
(a) isolated and purified human ASP having the amino acid sequence of the mature protein encoded by the DNA shown in Figure 2, having an amino terminus at position 201 of said figure or encoded by a naturally occurring allelic variant thereof;
(b) isolated and purified human ASP having the amino acid sequence of the mature protein encoded by the DNA shown in Figure 5 or Figure 6, and having a molecular weight of approximately 5 kd, or encoded by a naturally occurring allelic variant thereof; or
(c) isolated and purified canine ASP having the amino acid sequence of the mature protein encoded by the DNA shown in Figure 13, having a molecular weight of approximately 5 kd, or encoded by a naturally occurring allelic variant thereof;
or a corresponding cDNA which DNA is free of DNA encoding proteins normally accompanying the ASP.

2. A process for producing recombinant host cells comprising transforming them with an expression system comprising a recombinant DNA sequence which is
(a) isolated and purified human ASP having the amino acid sequence of the mature protein encoded by the DNA shown in Figure 2, having an amino terminus at position 201 of said figure or encoded by a naturally occurring allelic variant thereof;
(b) isolated and purified human ASP having the amino acid sequence of the mature protein encoded by the DNA shown in Figure 5 or Figure 6, and having a molecular weight of approximately 5 kd, or encoded by a naturally occurring allelic variant thereof; or
(c) isolated the purified canine ASP having the amino acid sequence of the mature protein encoded by the DNA shown in Figure 13, having a molecular weight of approximately 5 kd, or encoded by a naturally occurring allelic variant thereof
which DNA is free of DNA encoding proteins normally accompanying the ASP, and which DNA sequence is operably linked to control sequences effective in expressing said sequence in the recombinant host cells.

3. A process according to claim 1 or 2 in which the recombinant DNA sequence has the sequence shown in Figure 2, having an amino terminus at position 201 of said figure, Figure 5, Figure 6 or Figure 13.

4. A process according to claim 1, 2 or 3 in which the recombinant DNA is human or canine SP-5 DNA.

5. A process according to any one of claims 1 to 4 in which the recombinant DNA is human DNA.

6. A process for producing ASP comprising culturing the cells produced by the process of any one of claims 1 to 5.

7. A process for producing a pharmaceutical composition effective in treating respiratory distress syndrome (RDS) in mammals which process comprises admixing a protein produced according to claim 6 with a phospholipid preparation and, optionally, with a pharmaceutically acceptable excipient.

8. A process according to claim 7 which further comprises admixing with an effective amount of 32K ASP protein.

9. A process according to claim 7 or 8 for producing a composition which comprises from 50% to almost 100% by weight phospholipid.

10. A process according to any one of claims 7, 8 or 9 which comprises admixing with dipalmitoyl phosphatidyl choline (DPPC).

## Patentansprüche (Patentansprüche für folgende(n) Vertragsstaat(en): BE, CH, DE, FR, GB, IT, LI, LU, NL, SE)

1. Alveolar-oberflächenaktives Protein (ASP), das frei ist von Proteinen, die es normalerweise in situ begleiten, und das
a) isoliertes und gereinigtes menschliches ASP ist, mit der Aminosäuresequenz des ausgereiften Proteins, kodiert durch die in Figur 2 dargestellte DNA, mit einem Amino-Ende bei Position 201 dieser Figur, oder kodiert durch eine natürlich auftretende allelische Variante davon;
b) isoliertes und gereinigtes menschliches ASP ist, mit der Aminosäuresequenz des ausgereiften Proteins, kodiert durch die in Figur 5 oder Figur 6 dargestellte DNA, und mit einem Molekulargewicht von ca. 5 kd, oder kodiert durch eine natürlich auftretende allelische Variante davon; oder
c) isoliertes und gereinigtes Hunde-ASP ist, mit der Aminosäuresequenz des Proteins, kodiert durch die in Figur 13 angegebene DNA, und mit einem Molekulargewicht von ca. 5 kd, oder kodiert durch eine natürlich auftretende allelische Variante davon.

2. Protein nach Anspruch 1, dadurch gekennzeichnet, daß es durch die in Figur 2 (mit einem Amino-Ende an Position 201 dieser Figur), Figur 5, Figur 6 oder Figur 13 dargestellte DNA-Sequenz kodiert ist.

3. Protein nach Anspruch 1 oder 2, dadurch gekennzeichnet, daß es durch menschliche oder Hunde SP-5 DNA kodiert ist.

4. Protein nach Anspruch 1, 2 oder 3, dadurch gekennzeichnet, daß es durch menschliche DNA kodiert ist.

5. Rekombinanter Expressionsvektor umfassend eine DNA-Sequenz, die für das in einem der Ansprüche 1 bis 4 beanspruchte Protein kodiert, wobei die DNA frei ist von DNA, die Proteine kodiert, die normalerweise die Proteine nach Anspruch 1 begleiten, und das funktionsfähig an Kontrollsequenzen gebunden ist, die wirksam sind um diese Sequenz in geeigneten rekombinanten Wirtszellen zu exprimieren.

6. Rekombinante Wirtszellen, dadurch gekennzeichnet, daß sie mit dem Expressionsvektor gemäß Anspruch 5 transformiert sind.

7. Verfahren zur Herstellung von ASP, dadurch gekennzeichnet, daß man die Zellen nach Anspruch 6 kultiviert.

8. Pharmazeutische Zusammensetzung zur Behandlung des Atemnotsyndroms (RDS) in Säugern, dadurch gekennzeichnet, daß die Zusammensetzung ein Protein gemäß einem der Ansprüche 1 bis 4 in Mischung mit einer Phosphorlipidzusammensetzung und gegebenenfalls einem pharmazeutisch annehmbaren Träger umfaßt.

9. Zusammensetzung nach Anspruch 8, dadurch gekennzeichnet, daß sie außerdem eine wirksame Menge von 32K ASP-Protein umfaßt.

10. Zusammensetzung nach Anspruch 8 oder 9, dadurch gekennzeichnet, daß sie 50 bis fast 100 Gew.-% Phospholipid umfaßt.

11. Zusammensetzung nach einem der Ansprüche 8, 9 oder 10, dadurch gekennzeichnet, daß sie Dipalmitoylphosphatidylcholin (DPPC) umfaßt.

## Patentansprüche (Patentansprüche für folgende(n) Vertragsstaat(en): AT, ES, GR)

1. Verfahren zur Herstellung rekombinanter Wirtszellen, dadurch gekennzeichnet, daß man sie mit einer rekombinanten DNA-Sequenz transformiert, die
a) isoliertes und gereinigtes menschliches ASP ist, mit der Aminosäuresequenz von ausgereiftem Proteins, kodiert durch die in Figur 2 dargestellte DNA, mit einem Amino-Ende an Position 201 dieser Figur, oder kodiert durch eine natürlich auftretende allelische Variante davon;
b) isoliertes und gereinigtes menschliches ASP ist, mit der Aminosäuresequenz des ausgereiften Proteins, kodiert durch die in Figur 5 oder Figur 6 angegebene DNA, und mit einem Molekulargewicht von ca. 5 kd, oder kodiert durch eine natürlich auftretende allelische Variante davon; oder
c) isoliertes und gereinigtes Hunde-ASP ist, mit der Aminosäuresequenz des ausgereiften Proteins, kodiert durch die in Figur 13 angegebene DNA, und mit einem Molekulargewicht von ca. 5 kd, oder kodiert durch eine natürlich auftretende allelische Variante davon;
oder eine korrespondierende cDNA, welche DNA frei ist von DNA, die das ASP normalerweise begleitende Proteine kodiert.

2. Verfahren zur Herstellung rekombinanter Wirtszellen, dadurch gekennzeichnet, daß man sie mit einem Expressionssystem transformiert, das eine rekombinante DNA-Sequenz umfaßt, die
a) isoliertes und gereinigtes menschliches ASP ist, mit der Aminosäuresequenz des aus gereiften Proteins besitzt, kodiert durch die in Figur 2 dargestellte DNA, mit einem Amino-Ende an Position 201 dieser Figur, oder kodiert durch eine natürlich auftretende allelische Variante davon;
b) isoliertes und gereinigtes menschliches ASP ist, mit der Aminosäuresequenz des ausgereiften Proteins, kodiert durch die in Figur 5 oder Figur 6 angegebene DNA, und mit einem Molekulargewicht von ca. 5 kd, oder kodiert durch eine natürlich auftretende allelische Variante davon; oder
c) isoliertes und gereinigtes Hunde-ASP ist, mit der Aminosäuresequenz des ausgereiften Proteins, kodiert durch die in Figur 13 dargestellte DNA, mit einem Molekulargewicht von ca. 5 kd, oder kodiert durch eine natürlich auftretende allelische Variante davon,
wobei die DNA frei ist von DNA, die Proteine kodiert, die normalerweise das ASP begleiten, und wobei die DNA-Sequenz funktionell an Kontrollsequenzen gebunden ist, die wirksam sind um diese Sequenz in den rekombinanten Wirtszellen zu exprimieren.

3. Verfahren nach Anspruch 1 oder 2, dadurch gekennzeichnet, daß die rekombinante DNA-Sequenz die Sequenz besitzt, die dargestellt ist in Figur 2, mit einem Amino-Ende in Position 201 dieser Figur, Figur 5, Figur 6 oder Figur 13.

4. Verfahren nach Anspruch 1, 2 oder 3, daß die rekombinante DNA eine menschliche oder Hunde SP-5 DNA ist.

5. Verfahren nach einem der Ansprüche 1 bis 4, dadurch gekennzeichnet, daß die rekombinante DNA menschliche DNA ist.

6. Verfahren zur Herstellung von ASP, dadurch gekennzeichnet, daß man die nach dem Verfahren gemäß einem der Ansprüche 1 bis 5 hergestellten Zellen kultiviert.

7. Verfahren zur Herstellung einer pharmazeutischen Zusammensetzung zur Behandlung des Atemnotsyndroms (RDS) in Säugern, dadurch gekennzeichnet, daß man ein gemäß Anspruch 6 hergestelltes Protein mit einer Phospholipidzusammensetzung und gegebenenfalls mit einem pharmazeutisch annehmbaren Träger mischt.

8. Verfahren nach Anspruch 7, dadurch gekennzeichnet, daß es außerdem die Zumischung einer wirksamen Menge 32K ASP Protein umfaßt.

9. Verfahren nach Anspruch 7 oder 8, dadurch gekennzeichnet, daß man eine Zusammensetzung herstellt, die 50 bis fast 100 Gew.-% Phospholipid umfaßt.

10. Verfahren nach einem der Ansprüche 7, 8 oder 9, dadurch gekennzeichnet, daß man mit Dipalmitoylphosphatidylcholin (DPPC) mischt.

## Revendications (Revendications pour l'(les) Etat(s) contractant(s) suivant(s): BE, CH, DE, FR, GB, IT, LI, LU, NL, SE)

1. Protéine de surfactant alvéolaire (ASP) exempte de protéines qui l'accompagnent normalement in situ, qui est
(a) ASP humain isolé et purifié ayant la séquence d'acides aminés de la protéine mature codée par l'ADN présenté à la figure 2, ayant une extrémité aminoterminale en position 201 sur ladite figure, ou codée par un variant allélique de celui-ci survenant naturellement;
(b) ASP humain isolé et purifié ayant la séquence d'acides aminés de la protéine mature codée par l'ADN présenté à la figure 5 ou à la figure 6 et ayant un poids moléculaire d'approximativement 5 kd, ou codée par un variant allélique de celui-ci survenant naturellement; ou
(c) ASP canin isolé et purifié ayant la séquence d'acides aminés de la protéine mature codée par l'ADN présenté à la figure 13, ayant un poids moléculaire d'approximativement 5 kd, ou codée par un variant allélique de celui-ci survenant naturellement.

2. Protéine selon la revendication 1 qui est codée par la séquence d'ADN présentée à la figure 2 (ayant une extrémité aminoterminale en position 201 sur ladite figure), à la figure 5, à la figure 6 ou à la figure 13.

3. Protéine selon la revendication 1 ou 2 qui est codée par l'ADN de SP-5 humain ou canin.

4. Protéine selon les revendications 1, 2 ou 3 qui est codée par un ADN humain.

5. Vecteur d'expression recombinant comprenant une séquence d'ADN qui code pour la protéine revendiquée dans l'une quelconque des revendications 1 à 4, ADN qui est exempt d'ADN codant pour les protéines accompagnant normalement les protéines de la revendication 1, lié de manière fonctionnelle à des séquences régulatrices capables d'assurer l'expression de ladite séquence dans des cellules hôtes recombinantes appropriées.

6. Cellules hôtes recombinantes transformées avec le vecteur d'expression de la revendication 5.

7. Méthode de production d'ASP qui comprend la culture des cellules de la revendication 6.

8. Composition pharmaceutique efficace dans le traitement du syndrome de détresse respiratoire (SDR) chez des mammifères, composition qui comprend une protéine selon l'une quelconque des revendications 1 à 4 en mélange avec une préparation de phospholipides et, éventuellement, avec un excipient pharmaceutiquement acceptable.

9. Composition selon la revendication 8 qui comprend, en outre, une quantité efficace de la protéine ASP 32K.

10. Composition selon la revendication 8 ou 9 qui comprend de 50 % à près de 100 % en poids de phospholipides.

11. Composition selon l'une quelconque des revendications 8, 9 ou 10 qui comprend de la dipalmitoyl-phosphatidylcholine (DPPC).

## Revendications (Revendications pour l'(les) Etat(s) contractant(s) suivant(s): AT, ES, GR)

1. Procédé de production de cellules hôtes recombinantes comprenant leur transformation avec une séquence d'ADN recombinant qui est
(a) ASP humain isolé et purifié ayant la séquence d'acides aminés de la protéine mature codée par l'ADN présenté à la figure 2, ayant une extrémité aminoterminale en position 201 sur ladite figure, ou codée par un variant allélique de celui-ci survenant naturellement;
(b) ASP humain isolé et purifié ayant la séquence d'acides aminés de la protéine mature codée par l'ADN présenté à la figure 5 ou à la figure 6 et ayant un poids moléculaire d'approximativement 5 kd, ou codée par un variant allélique de celui-ci survenant naturellement; ou
(c) ASP canin isolé et purifié ayant la séquence d'acides aminés de la protéine mature codée par l'ADN présenté à la figure 13, ayant un poids moléculaire d'approximativement 5 kd, ou codée par un variant allélique de celui-ci survenant naturellement ;
ou un ADNc correspondant, ADN qui est exempt d'ADN codant pour les protéines accompagnant normalement l'ASP.

2. Procédé de production de cellules hôtes recombinantes comprenant leur transformation avec un système d'expression comprenant une séquence d'ADN recombinant qui est
(a) ASP humain isolé et purifié ayant la séquence d'acides aminés de la protéine mature codée par l'ADN présenté à la figure 2, ayant une extrémité aminoterminale en position 201 sur ladite figure, ou codée par un variant allélique de celui-ci survenant naturellement ;
(b) ASP humain isolé et purifié ayant la séquence d'acides aminés de la protéine mature codée par l'ADN présenté à la figure 5 ou à la figure 6 et ayant un poids moléculaire d'approximativement 5 kd, ou codée par un variant allélique de celui-ci survenant naturellement; ou
(c) ASP canin isolé et purifié ayant la séquence d'acides aminés de la protéine mature codée par l'ADN présenté à la figure 13, ayant un poids moléculaire d'approximativement 5 kd, ou codée par un variant allélique de celui-ci survenant naturellement ,
ADN qui est exempt d'ADN codant pour les protéines accompagnant normalement l'ASP, et séquence d'ADN qui est liée de manière fonctionnelle à des séquences régulatrices capables d'assurer l'expression de ladite séquence dans les cellules hôtes recombinantes.

3. Procédé selon la revendication 1 ou 2 dans lequel la séquence d'ADN recombinant possède la séquence présentée à la figure 2, ayant une extrémité aminoterminale en position 201 sur ladite figure, à la figure 5, à la figure 6 ou à la figure 13.

4. Procédé selon la revendication 1, 2 ou 3 dans lequel l'ADN recombinant est l'ADN de SP-5 humain ou canin.

5. Procédé selon l'une quelconque des revendications 1 à 4, dans lequel l'ADN recombinant est l'ADN humain.

6. Procédé de production d'ASP comprenant la culture des cellules produites par le procédé de l'une quelconque des revendications 1 à 5.

7. Procédé de production d'une composition pharmaceutique efficace dans le traitement du syndrome de détresse respiratoire (SDR) chez des mammifères, procédé qui comprend le mélange d'une protéine produite selon la revendication 6 avec une préparation de phospholipides et, éventuellement, avec un excipient pharmaceutiquement acceptable.

8. Procédé selon la revendication 7 qui comprend en outre le mélange avec une quantité efficace de protéine ASP 32K.

9. Procédé selon la revendication 7 ou 8 pour la production d'une composition qui comprend entre 50 % et près de 100 % en poids de phospholipides.

10. Procédé selon l'une quelconque des revendications 7, 8 ou 9 qui comprend le mélange avec de la dipalmitoyl-phosphatidylcholine (DPPC).
